# EUROPEAN PATENT APPLICATION

(11) **EP 4 484 580 A1**
(43) Date of publication of application: **01.01.2025**
(21) Application number: 22926520.2
(22) Date of filing: 21.02.2022
(51) Int. Cl.: C12Q 1/6895, C12Q 1/6851, C12Q 1/6813, C12N 15/11, C12N 5/10, C12N 15/32, C12N 15/54, C12N 15/82, A01H 5/00, A01H 6/54, A01G 22/40, A01G 13/00, A01H 1/02, A23L 11/00, A23L 11/45, A23J 1/14, A23D 9/00, A23L 29/30, A23L 29/00

(54) **NUCLEIC ACID SEQUENCE FOR DETECTING GLYCINE MAX PLANT DBN8205 AND DETECTION METHOD THEREFOR**

(71) Applicant: Beijing Dabeinong Biotechnology Co., Ltd., Beijing 100194 (CN)
(72) Inventor: YU, Caihong, Beijing 100193 (CN); XIE, Xiangting, Beijing 100193 (CN); DI, Shaokang, Beijing 100193 (CN); HAN, Chao, Beijing 100193 (CN); LI, Yunting, Beijing 100193 (CN); ZHANG, Linlin, Beijing 100193 (CN); BAO, Xiaoming, Beijing 100193 (CN)
(74) Representative: Nederlandsch Octrooibureau
(86) International application number: PCT/CN2022/077093
(87) International publication number: WO 2023/155193

(57) **Abstract**

Provided are a nucleic acid sequence for detecting a Glycine max plant DBN8205 and a detection method therefor. The nucleic acid sequence comprises SEQ ID NO: 1 or a complementary sequence thereof, and/or SEQ ID NO: 2 or a complementary sequence thereof. The Glycine max plant DBN8205 has good resistance to insects of Lepidoptera order and good tolerance to glufosinate herbicides, and therefore the yield is not affected. By utilizing the detection method, it is possible to accurately and quickly identify whether a biological sample contains a DNA molecule of a transgenic Glycine max event DBN8205.

## Description

### FIELD OF THE INVENTION

The present invention relates to the field of plant molecular biology, especially the field of transgenic crop breeding in the agricultural biotechnology research. In particular, the present invention relates to an insect resistant and glufosinate ammonium herbicide tolerant transgenic soybean event DBN8205, nucleic acid sequences for detecting whether a biological sample contains the specific transgenic soybean event DBN8205 and detection methods thereof.

### BACKGROUND

Soybean (*Glycine max*) is one of the five main crops in the world. Biotechnology methods have been applied to such crops to produce soybean varieties with desirable traits. The two most important agronomic traits in soybean production are insect resistance and herbicide tolerance. The insect resistance of soybean may be conferred by expressing insect resistance genes in soybean plants through a transgenic method, and transgenic soybeans that rely on expression of a single insect-resistant protein against insect infestation are at risk of limited tolerance, since insects will evolve resistance to insecticidal proteins expressed in transgenic soybeans under constant selective pressure, which once developed and not effectively controlled will undoubtedly limit the commercial value of insecticidal protein-containing transgenic soybean varieties. Therefore, the combined use of two or more insecticidal proteins can be used as a method for delaying insect resistance, and at the same time can broaden the insect-resistant spectrum. The phosphinothricin N-acetyltransferase (PAT) isolated from Streptomyces catalyses the conversion of L-phosphinothricin to its inactive form by acetylation. The plant-optimized forms of genes expressing PAT have been used in soybeans to confer tolerance to glufosinate ammonium herbicides, such as soybean event A5547-127.

It is of great significance to design an expression vector containing exogenous functional genes (cCry2Ab, cCry1Ac and cPAT genes) suitable for transforming soybean crops and to obtain the corresponding commercial transgenic soybean events. In addition to the functional genes (cCry2Ab, cCry1Ac and cPAT genes) themselves, the selection of regulatory elements is crucial to obtain a good transformation event and the technical effect is unpredictable. For example, the commercial glyphosate-resistant soybean transformation events GTS 40-3-2 (US5633435) and MON89788 (CN101252831B), both of which have been transformed with the CP4-EPSPS gene (the amino acid sequences are the same) having the molecular design of a single expression cassette, but due to the selection of different regulatory elements, have significantly different expression levels of EPSPS protein and different yield levels. Therefore, the combination and interaction of regulatory elements and the arrangement design on the T-DNA need to be fully considered and analyzed in the design of expression vectors. Meanwhile, good commercially suitable soybean transformation event also requires a comprehensive consideration of factors such as vector design for the cCry2Ab, cCry1Ac and cPAT genes in soybean plants, interactions between the three expression cassettes, insect resistance efficacy, herbicide tolerance efficacy, and the impacts on yield and other plant physiological indices, so that the cCry2Ab, cCry1Ac and cPAT genes can be expressed in appropriate amounts in soybean and serve their corresponding functions without influencing the yield and other physiological indices of the soybean event.

The expression of exogenous genes in plants is known to be influenced by their positions on chromosomes, perhaps due to chromatin structure (e.g., heterochromatin) or the proximity of transcriptional regulation elements (e.g., enhancer) to the integration site. For this reason, it is often necessary to screen a large number of events in order to identify the event that can be commercialized (i.e. the event in which an introduced target gene is optimally expressed). For example, it has been observed in plants and other organisms that there may be great difference among events in term of the expression level of an introduced gene; and there may also be differences in term of spatial or temporal patterns of expression, for example, differences in the relative expression of a transgene among different plant tissues, which are reflected in the possible discrepancy between the actual expression pattern and the expected expression pattern based on transcriptional regulatory elements in the introduced gene construct. For this reason, it usually needs to produce hundreds to thousands of different events and screen those events for a single event that has expected transgene expression level and pattern for commercial purposes. An event that has expected expression levels and patterns of a transgene is useful for introgression of the transgene into other genetic backgrounds by sexual outcross using conventional breeding methods. The progeny produced by such crossing maintains the transgene expression characteristics of the original transformant. This strategy is used to ensure reliable gene expression in a number of varieties that are well adapted to local growing conditions.

It would be advantageous to be able to detect the presence of a particular event in order to determine whether the progeny of a sexual cross contains a target gene. In addition, the method for detecting a particular event would be helpful for complying with the related regulations such as those requiring premarket approval and labeling of the foods derived from recombinant crops. It is possible to detect the presence of a transgene by any well-known methods for detecting a polynucleotide, such as polymerase chain reaction (PCR) or DNA hybridization using polynucleotide probes. These detection methods generally focus on frequently used genetic elements, such as promoters, terminators, marker genes. As a result, such methods may not be useful for distinguishing different events, particularly those produced by using the same DNA construct, unless the sequence of the chromosomal DNA ("flanking DNA") adjacent to the inserted transgenic DNA is known. Thus, a pair of primers spanning the junction between the inserted transgene and the flanking DNA is usually used to identify a particular transgenic event by PCR, in particular, a first primer comprised in the inserted sequence and a second primer comprised in the inserted sequence.

### SUMMARY OF THE INVENTION

The object of the present invention is to provide nucleic acid sequences for detecting soybean plant DBN8205 and detection methods thereof. The transgenic soybean event DBN8205 has good insect resistance as well as good glufosinate ammonium herbicide tolerance. The detection methods can accurately and rapidly identify whether a biological sample contains a DNA molecule of the transgenic soybean event DBN8205.

To achieve the above object, the present invention provides a nucleic acid molecule having the following nucleic acid sequences: said nucleic acid sequence comprises at least 11 consecutive nucleotides at positions 1-462 of SEQ ID NO: 3 or a complementary sequence thereof, and at least 11 consecutive nucleotides at positions 463-634 of SEQ ID NO: 3 or a complementary sequence thereof; and/or at least 11 consecutive nucleotides at positions 1-225 of SEQ ID NO: 4 or a complementary sequence thereof, and at least 11 consecutive nucleotides at positions 226-642 of SEQ ID NO: 4 or a complementary sequence thereof.

Preferably, said nucleic acid sequence comprises 22-25 consecutive nucleotides at positions 1-462 of SEQ ID NO: 3 or a complementary sequence thereof, and 22-25 consecutive nucleotides at positions 463-634 of SEQ ID NO: 3 or a complementary sequence thereof; and/or 22-25 consecutive nucleotides at positions 1-225 of SEQ ID NO: 4 or a complementary sequence thereof, and 22-25 consecutive nucleotides at positions 226-642 of SEQ ID NO: 4 or a complementary sequence thereof.

Preferably, said nucleic acid sequence comprises SEQ ID NO: 1 or a complementary sequence thereof, and/or SEQ ID NO: 2 or a complementary sequence thereof.

Said SEQ ID NO: 1 or a complementary sequence thereof is a sequence of 22 nucleotides that is adjacent to the insertion junction at the 5' end of the inserted sequence in the transgenic soybean event DBN8205. Said SEQ ID NO: 1 or a complementary sequence thereof spans the genomic DNA sequences flanking the soybean insertion site and the DNA sequence at the 5' end of the inserted sequence. Therefore, inclusion of SEQ ID NO: 1 or a complementary sequence thereof could be identified as the presence of the transgenic soybean event DBN8205. Said SEQ ID NO: 2 or a complementary sequence thereof is a sequence of 22 nucleotides that is adjacent to the insertion junction at the 3' end of the inserted sequence in the transgenic soybean event DBN8205. Said SEQ ID NO: 2 or a complementary sequence thereof spans the DNA sequence at the 3' end of the inserted sequence and the genomic DNA sequences flanking the soybean insertion site. Therefore, inclusion of SEQ ID NO: 2 or a complementary sequence thereof could be identified as the presence of the transgenic soybean event DBN8205.

Preferably, said nucleic acid sequence comprises SEQ ID NO: 3 or a complementary sequence thereof, and/or SEQ ID NO: 4 or a complementary sequence thereof.

Said nucleic acid sequence of the present invention comprises at least 11 or more consecutive polynucleotides in any portion of the T-DNA inserted sequence in SEQ ID NO: 3 or a complementary sequence thereof (the first nucleic acid sequence), and at least 11 or more consecutive polynucleotides in any portion of the 5' flanking soybean genomic DNA region in SEQ ID NO: 3 or a complementary sequence thereof (the second nucleic acid sequence). Further, said nucleic acid sequence can be homologous or complementary to a portion of SEQ ID NO: 3 comprising the entire SEQ ID NO: 1. When used together, the first nucleic acid sequence and the second nucleic acid sequence can be used as a DNA primer pair in a DNA amplification method that produces an amplification product. If the amplification product produced by using a DNA primer pair in the DNA amplification method comprises SEQ ID NO: 1, the presence of the transgenic soybean event DBN8205 or progeny thereof can be diagnosed. Said SEQ ID NO: 3 or a complementary sequence thereof is a sequence of 634 nucleotides that is adjacent to the insertion junction at the 5' end of the T-DNA inserted sequence in the transgenic soybean event DBN8205. SEQ ID NO: 3 or a complementary sequence thereof consists of 462 nucleotides from the soybean genomic 5' flanking sequence (nucleotides 1-462 of SEQ ID NO: 3) and 172 nucleotides from pDBN4031 construct DNA sequence (nucleotides 463-634 of SEQ ID NO: 3). Therefore, inclusion of SEQ ID NO: 3 or a complementary sequence thereof could be identified as the presence of the transgenic soybean event DBN8205.

Said nucleic acid sequence comprises at least 11 or more consecutive polynucleotides in any portion of the T-DNA inserted sequence in SEQ ID NO: 4 or a complementary sequence thereof (the third nucleic acid sequence), and at least 11 or more consecutive polynucleotides in any portion of the 3' flanking soybean genomic DNA region in SEQ ID NO: 4 or a complementary sequence thereof (the fourth nucleic acid sequence). Further, said nucleic acid sequence can be homologous or complementary to a portion of SEQ ID NO: 4 comprising the entire SEQ ID NO: 2. When used together, the third nucleic acid sequence and the fourth nucleic acid sequence can used as a DNA primer pair in a DNA amplification method that produces an amplification product. If the amplification product produced by using said DNA primer pair in the DNA amplification method comprises SEQ ID NO: 2, the presence of the transgenic soybean event DBN8205 or progeny thereof can be diagnosed. Said SEQ ID NO: 4 or a complementary sequence thereof is a sequence of 642 nucleotides that is adjacent to the T-DNA insertion junction at the 3' end of the inserted sequence in the transgenic soybean event DBN8205. SEQ ID NO: 4 or a complementary sequence thereof consists of 21 nucleotides from the DNA sequence of a t35S transcriptional terminator (nucleotides 1-21 of SEQ ID NO:4), 204 nucleotides from pDBN4031 construct DNA sequence (nucleotides 22-225 of SEQ ID NO: 4), and 417 nucleotides from the soybean genomic 3' flanking sequence (nucleotides 226-642 of SEQ ID NO: 4). Therefore, inclusion of SEQ ID NO: 4 or a complementary sequence thereof could be identified as the presence of the transgenic soybean event DBN8205.

Further, said nucleic acid sequence comprises SEQ ID NO: 5 or a complementary sequence thereof.

Said SEQ ID NO: 5 or a complementary sequence thereof is a sequence of 12813 nucleotides that characterizes the transgenic soybean event DBN8205. The specific genomes and genetic elements contained in SEQ ID NO: 5 are shown in Table 1. Inclusion of SEQ ID NO: 5 or a complementary sequence thereof could be identified as the presence of the transgenic soybean event DBN8205.

**Table 1: The genomes and genetic elements contained in SEQ ID NO: 5**

| Genetic element/genome | Length (bp) | Position in SEQ ID NO: 5 |
|---|---|---|
| 5' genome | 481 | 1-481 |
| RB | 137 | 482-618 |
| prAtAct2-01 | 1408 | 866-2273 |
| spAtCTP2 | 228 | 2282-2509 |
| cCry2Ab | 1905 | 2519-4423 |
| tPsE9 | 643 | 4431-5073 |
| prAtRbcS4 | 1723 | 5080-6802 |
| spAtRbcS4 | 264 | 6803-7066 |
| cCry1Ac | 3537 | 7067-10603 |
| tNos | 253 | 10610-10862 |
| pr35S | 530 | 10916-11445 |
| cPAT | 552 | 11446-11997 |
| t35S | 195 | 11998-12192 |
| LB | 50 | 12347-12396 |
| 3' genome | 417 | 12397-12813 |

As is well known to those skilled in the art, the first, second, third and fourth nucleic acid sequences may not necessarily consist of DNA alone, but may also comprise RNA, a mixture of DNA and RNA, or a combination of DNA, RNA or other nucleotides or analogues thereof that do not act as templates for one or more polymerases. In addition, the probes or primers of the present invention should be at least about 11, 12, 13, 14, 15, 16, 17, 18, 19, 20, 21 or 22 consecutive nucleotides in length and may be selected from the nucleotides as set forth in SEQ ID NO: 1, SEQ ID NO: 2, SEQ ID NO: 3, SEQ ID NO: 4 and SEQ ID NO: 5. When selected from the nucleotides as set forth in SEQ ID NO: 3, SEQ ID NO: 4 and SEQ ID NO: 5, the probes and primers may be at least about 21 to about 50 or more consecutive nucleotides in length.

Said nucleic acid sequences or complementary sequences thereof can be used in DNA amplification methods to produce amplicons which are used to detect the presence of the transgenic soybean event DBN8205 or progeny thereof in a biological sample. Said nucleic acid sequences or complementary sequences thereof can be used in nucleotide detection methods to detect the presence of the transgenic soybean event DBN8205 or progeny thereof in a biological sample.

To achieve the above object, the present invention also provides a method for detecting the presence of DNA of the transgenic soybean event DBN8205 in a sample, comprising:
- contacting the sample to be detected with at least two primers for amplifying a target amplification product in a nucleic acid amplification reaction;
- performing the nucleic acid amplification reaction; and
- detecting the presence of the target amplification product;
wherein the target amplification product comprises said nucleic acid sequences.

Preferably, the target amplification product comprises SEQ ID NO: 1 or a complementary sequence thereof, SEQ ID NO: 2 or a complementary sequence thereof, SEQ ID NO: 6 or a complementary sequence thereof, and/or SEQ ID NO: 7 or a complementary sequence thereof.

In particular, the two primers comprise SEQ ID NO: 8 and SEQ ID NO: 9, SEQ ID NO: 10 and SEQ ID NO: 11, or the complementary sequences of SEQ ID NO: 1 and SEQ ID NO: 2.

To achieve the above object, the present invention also provides a method for detecting the presence of DNA of the transgenic soybean event DBN8205 in a sample, comprising:
- contacting the sample to be detected with a probe, wherein the probe comprises said nucleic acid sequences;
- hybridizing the sample to be detected with the probe under stringent hybridization conditions; and
- detecting the hybridization of the sample to be detected with the probe.

The stringent conditions may be hybridization at 65°C in a solution of 6 × SSC (sodium citrate) and 0.5% SDS (sodium dodecyl sulfate), followed by membrane washing in a solution of 2 × SSC and 0.1% SDS and a solution of 1 × SSC and 0.1% SDS (each for one time).

Preferably, the probe comprises SEQ ID NO: 1 or a complementary sequence thereof, SEQ ID NO: 2 or a complementary sequence thereof, SEQ ID NO: 6 or a complementary sequence thereof, and/or SEQ ID NO: 7 or a complementary sequence thereof.

Optionally, at least one of the probes is labeled with at least one fluorophore.

To achieve the above object, the present invention also provides a method for detecting the presence of DNA of the transgenic soybean event DBN8205 in a sample, comprising:
- contacting the sample to be detected with a marker nucleic acid molecule, wherein the marker nucleic acid molecule comprises said nucleic acid sequences;
- hybridizing the sample to be detected with the marker nucleic acid molecule under stringent hybridization conditions;
- detecting the hybridization of the sample to be detected with the marker nucleic acid molecule, and further performing a marker-assisted breeding analysis for determining whether the insect resistance and/or herbicide tolerance is genetically linked to the marker nucleic acid molecule.

Preferably, the marker nucleic acid molecule comprises at least one sequence selected from the group consisting of SEQ ID NO: 1 or a complementary sequence thereof, SEQ ID NO: 2 or a complementary sequence thereof, and SEQ ID NOs: 6-11 or complementary sequences thereof.

To achieve the above object, the present invention also provides a DNA detection kit comprising at least one DNA molecule, wherein the DNA molecule comprises said nucleic acid sequence, and can act as a DNA primer or a probe specific for the transgenic soybean event DBN8205 or progeny thereof.

Preferably, the DNA molecule comprises SEQ ID NO: 1 or a complementary sequence thereof, SEQ ID NO: 2 or a complementary sequence thereof, SEQ ID NO: 6 or a complementary sequence thereof, and/or SEQ ID NO: 7 or a complementary sequence thereof.

To achieve the above object, the present invention also provides a plant cell or part comprising a nucleic acid sequence encoding the insect resistant Cry2Ab protein, a nucleic acid sequence encoding the insect resistant Cry1Ac protein, a nucleic acid sequence encoding the glufosinate ammonium tolerant PAT protein and a nucleic acid sequence of a specific region, wherein the nucleic acid sequence of the specific region comprises the sequence as set forth in SEQ ID NO: 1 and/or SEQ ID NO: 2; preferably, the nucleic acid sequence of the specific region comprises the sequence as set forth in SEQ ID NO: 3 and/or SEQ ID NO: 4.

Preferably, the plant cell or part successively comprises SEQ ID NO: 1, the nucleic acid sequence at positions 866-12192 of SEQ ID NO: 5 and SEQ ID NO: 2, or comprises the sequence as set forth in SEQ ID NO: 5.

Preferably, the plant cell or part comprises the transgenic soybean event DBN8205;
Optionally, the plant cell or part further comprises at least one other transgenic soybean event different from transgenic soybean event DBN8205; preferably, the other transgenic soybean event is transgenic soybean event DBN9004 and/or transgenic soybean event DBN8002.

To achieve the above object, the present invention also provides a method for protecting a soybean plant from insect invasion, comprising providing at least one transgenic soybean plant cell in the diet of the target insect, wherein the transgenic soybean plant cell comprises in its genome the sequence as set forth in SEQ ID NO: 1 and/or SEQ ID NO: 2; and ingestion of the transgenic soybean plant cell inhibits the target insect from further ingesting the transgenic soybean plant.

Preferably, the transgenic soybean plant cell comprises in its genome the sequence as set forth in SEQ ID NO: 3 and/or SEQ ID NO: 4.

Preferably, the transgenic soybean plant cell successively comprises in its genome SEQ ID NO: 1, the nucleic acid sequence at positions 866-12192 of SEQ ID NO: 5 and SEQ ID NO: 2, or comprises SEQ ID NO: 5.

To achieve the above object, the present invention also provides a method for protecting a soybean plant from damage caused by a herbicide or controlling weeds in a field in which a soybean plant is planted, comprising applying an effective amount of glufosinate ammonium herbicide into the field in which at least one transgenic soybean plant is planted, wherein the transgenic soybean plant comprises in its genome the sequence as set forth in SEQ ID NO: 1 and/or SEQ ID NO: 2, and the transgenic soybean plant has glufosinate ammonium herbicide tolerance.

Preferably, the transgenic soybean plant comprises in its genome the sequence as set forth in SEQ ID NO: 3 and/or SEQ ID NO: 4.

Preferably, the transgenic soybean plant successively comprises in its genome SEQ ID NO: 1, the nucleic acid sequence at positions 866-12192 of SEQ ID NO: 5 and SEQ ID NO: 2, or comprises the sequence as set forth in SEQ ID NO: 5.

To achieve the above object, the present invention also provides a method for breeding an insect resistant and/or glufosinate ammonium herbicide tolerant soybean plant, comprising:
- planting at least one soybean seed, wherein the soybean seed comprises in its genome a nucleic acid sequence encoding the insect resistant Cry2Ab protein and/or a nucleic acid sequence encoding the insect resistant Cry1Ac protein and/or a nucleic acid sequence encoding the glufosinate ammonium herbicide tolerant PAT protein, and a nucleic acid sequence of a specific region, or the soybean seed comprises in its genome the nucleic acid sequence as set forth in SEQ ID NO: 5;
- growing the soybean seed into a soybean plant; and
- invading the soybean plant with a target insect, and/or spraying the soybean plant with an effective amount of glufosinate ammonium herbicide, and then harvesting the plant with reduced plant damage as compared with other plants which do not have the nucleic acid sequence of the specific region;
wherein the nucleic acid sequence of the specific region is the sequence as set forth in SEQ ID NO: 1 and/or SEQ ID NO: 2; preferably, the nucleic acid sequence of the specific region is the sequence as set forth in SEQ ID NO: 3 and/or SEQ ID NO: 4.

To achieve the above object, the present invention also provides a method for producing an insect resistant and/or glufosinate ammonium herbicide tolerant soybean plant, comprising:
- introducing a nucleic acid sequence encoding the insect resistant Cry2Ab protein and/or a nucleic acid sequence encoding the insect resistant Cry1Ac protein and/or a nucleic acid sequence encoding the glufosinate ammonium herbicide tolerant PAT protein, and a nucleic acid sequence of a specific region comprised in the genome of a first soybean plant, or the nucleic acid sequence as set forth in SEQ ID NO: 5 comprised in the genome of a first soybean plant, into a second soybean plant, thereby producing a plurality of progeny plants; and
- selecting the progeny plants comprising the nucleic acid sequence of the specific region, which are insect resistant and/or glufosinate ammonium herbicide tolerant;
wherein the nucleic acid sequence of the specific region is the sequence as set forth in SEQ ID NO: 1 and/or SEQ ID NO: 2; preferably, the nucleic acid sequence of the specific region is the sequence as set forth in SEQ ID NO: 3 and/or SEQ ID NO: 4.

Preferably, the method comprises: sexually crossing a first soybean plant comprising the transgenic soybean event DBN8205 with a second soybean plant, thereby producing a plurality of progeny plants; selecting the progeny plants comprising the nucleic acid sequence of the specific region;
- invading the progeny plants with a target insect, and/or treating the progeny plants with glufosinate ammonium; and
- selecting the progeny plants which are resistant to the target insect and/or tolerant to glufosinate ammonium herbicide.

To achieve the above object, the present invention also provides an agricultural product or commodity derived from a soybean plant comprising the transgenic soybean event DBN8205, wherein the agricultural product or commodity is lecithin, fatty acids, glycerol, sterols, soy flakes, soy flours, soy proteins or their concentrates, soybean oils, soy protein fibers, soy milk clots or bean curd.

To achieve the above object, the present invention also provides an agricultural product or commodity derived from a soybean plant comprising the transgenic soybean event DBN8205, wherein the soybean plant further comprises at least one other transgenic soybean event different from transgenic soybean event DBN8205;
Preferably, the other transgenic soybean event is transgenic soybean event DBN9004 and/or transgenic soybean event DBN8002.

To achieve the above object, the present invention also provides a method for expanding the insect-resistant spectrum and/or the herbicide-tolerant range for a plant, wherein the transgenic soybean event DBN8205 is expressed in a plant together with at least one other transgenic soybean event different from transgenic soybean event DBN8205;
Preferably, the other transgenic soybean event is transgenic soybean event DBN9004 and/or transgenic soybean event DBN8002.

The DBN9004 provided by the present invention is the transgenic soybean event disclosed in the patent CN106086011A, wherein the transgenic soybean event DBN9004 is deposited in the form of seeds and under the accession number CGMCC No. 11171 at the China General Microbiological Culture Collection Center, CGMCC.

The DBN8002 provided by the present invention is the transgenic soybean event disclosed in the patent CN111406117A, wherein the transgenic soybean event DBN8002 is deposited in the form of seeds and under the accession number CGMCC No. 17299 at the China General Microbiological Culture Collection Center, CGMCC.

In the nucleic acid sequences for detecting soybean plants and detection methods thereof according to the present invention, the following definitions and methods are provided to better define the present invention and to guide those skilled in the art in the implementation of the present invention. Unless otherwise stated, the terms are to be understood according to conventional usage by those skilled in the art.

The term "soybean" refers to *Glycine max* and comprises all plant varieties that can mate with the soybean, including wild soybean species.

The term "including", "comprising" or "containing" means "comprising but not limited to".

The term "plant" includes the whole plants, plant cells, plant organs, plant protoplasts, plant cell tissue cultures from which plants can be regenerated, plant calli, plant clumps, and plant cells that are intact in plants or plant parts, wherein the plant parts can be, for example, embryos, pollens, ovules, seeds, leaves, flowers, branches, fruit, stalks, roots, root tips, or anthers. It is to be understood that within the scope of the present invention, parts of transgenic plants include but are not limited to plant cells, protoplasts, tissues, calli, embryos, flowers, stems, fruits, leaves and roots. The above-mentioned plant parts are derived from the transgenic plants or progeny thereof which have been previously transformed with the DNA molecules of the present invention and therefore at least partially consist of transgenic cells.

The term "gene" refers to a nucleic acid fragment that expresses a specific protein, including regulatory sequences preceding (5' non-coding sequences) and following (3' non-coding sequences) the coding sequence. "Native gene" refers to a gene with its own regulatory sequences as found in nature. "Chimeric gene" refers to any gene that is not a native gene, comprising regulatory and coding sequences that are not found in nature. "Endogenous gene" refers to a native gene at its natural location in the genome of an organism. "Exogenous gene" is a foreign gene currently present in the genome of an organism which does not contain it naturally, and also refers to a gene introduced into a receptor cell via a transgenic procedure. Exogenous genes can comprise native genes or chimeric genes inserted into a non-native organism. "Transgene" is a gene that has been introduced into a genome by a transformation procedure. "Insertion site" or "target site" refers to the site in a plant genome into which a recombinant DNA has been inserted.

"Flanking DNA" can comprise either a genome naturally present in an organism such as a plant, or an exogenous (heterologous) DNA introduced via a transformation procedure, e.g. a fragment associated with a transformation event. Thus, flanking DNA may include a combination of native DNA and exogenous DNA. As used herein, the term "flanking DNA", also called as "flanking region", "flanking sequence", "flanking genomic sequence", or "flanking genomic DNA", refers to a sequence of at least 3, 5, 10, 11, 15, 20, 50, 100, 200, 300, 400, 1000, 1500, 2000, 2500 or 5000 base pairs or greater, which is located either immediate upstream or downstream of the originally inserted exogenous DNA molecule and is adjacent to the DNA molecule. When this flanking region is located downstream, it may also be referred to as "3' flank" or "left border flank", and the like. When this flanking region is located upstream, it may also be referred to as "5' flank" or "right border flank", and the like.

Transformation procedures leading to random integration of an exogenous DNA will result in transformants containing different flanking regions which are specific for each transformant. When a recombinant DNA is introduced into a plant through traditional crossing, its flanking regions will generally not be changed. Transformants will also contain unique junctions between a heterologous insert DNA and a genomic DNA fragment, or between two genomic DNA fragments, or between two heterologous DNA fragments. "Junction" is a point where two specific DNA fragments are linked. For example, a junction exists in the position where an insert DNA is linked to a flanking DNA. A junction point also exists in a transformed organism in which two DNA fragments are linked together in a manner modified from that found in a native organism. "Junction region" or "junction sequence" refers to DNA that comprises a junction point.

The present invention provides a transgenic soybean event called DBN8205 and its progeny. The transgenic soybean event DBN8205 is also referred to as a soybean plant DBN8205, including plants and seeds of the transgenic soybean event DBN8205, together with plant cells or regenerable parts thereof, wherein the plant parts of the transgenic soybean event DBN8205 include but are not limited to cells, pollens, ovules, flowers, buds, roots, stems, leaves, pods and products derived from the soybean plant DBN8205 such as soybean cakes, powders and oils, specifically lecithin, fatty acids, glycerol, sterols, edible oils, defatted soy flakes, including defatted and baked soy flours, soy milk clots, bean curd, soy protein concentrates, isolated soy proteins, hydrolyzed vegetable proteins, organized soy proteins and soy protein fibers.

The transgenic soybean event DBN8205 of the present invention contains a DNA construct which, when expressed in plant cells, confers the transgenic soybean event DBN8205 resistance to insects and tolerance to glufosinate ammonium herbicide. The DNA construct comprises three expression cassettes arranged in tandem. The first expression cassette comprises a suitable promoter for expression in a plant, a nucleic acid sequence encoding a signal peptide/transit peptide, a nucleic acid sequence encoding a Cry2Ab protein and a suitable polyadenylation signal sequence, wherein the Cry2Ab protein is mainly resistant to *Lepidoptera* insects. The second expression cassette comprises a suitable promoter for expression in a plant, a nucleic acid sequence encoding a signal peptide/transit peptide, a nucleic acid sequence encoding a Cry1Ac protein and a suitable polyadenylation signal sequence, wherein the Cry 1 Ac protein is also mainly resistant to *Lepidoptera* insects. The third expression cassette comprises a suitable promoter for expression in a plant, a nucleic acid sequence encoding phosphinothricin N-acetyltransferase (PAT) and a suitable polyadenylation signal sequence, wherein the PAT protein is tolerant to glufosinate ammonium herbicide. Furthermore, the promoter may be a suitable promoter isolated from plants, including constitutive, inducible and/or tissue-specific promoters. The suitable promoter includes but is not limited to, Cauliflower mosaic virus (CaMV) 35S promoter, figwort mosaic virus (FMV) 35S promoter, Ubiquitin promoter, Actin promoter, *Agrobacterium tumefaciens* nopaline synthetase (NOS) promoter, octopine synthetase (OCS) promoter, *Cestrum* yellow leaf curl virus promoter, Patatin promoter, ribulose-1,5-bisphosphate carboxylase/oxygenase (RuBisCO) promoter, glutathione S-transferase (GST) promoter, E9 promoter, GOS promoter, alcA/alcR promoter, *Agrobacterium rhizogenes* RolD promoter and *Arabidopsis thaliana* Suc2 promoter. The signal peptide/transit peptide may direct Cry2Ab protein and/or Cry1Ac protein and transport them into a specific intracellular organelle or compartment. For example, a sequence encoding chloroplast transit peptide is used for targeting chloroplast, or a 'KDEL' retention sequence is used for targeting endoplasmic reticulum. The polyadenylation signal sequence may be a suitable polyadenylation signal sequence that works in plants. The suitable polyadenylation signal sequence includes but is not limited to, a polyadenylation signal sequence derived from nopaline synthetase (NOS) gene of *Agrobacterium tumefaciens,* a polyadenylation signal sequence derived from Cauliflower mosaic virus (CaMV) 35S terminator, a polyadenylation signal sequence derived from Protease Inhibitor II (PIN II) gene and a polyadenylation signal sequence derived from α-tubulin gene.

Moreover, the expression cassette may further comprise other genetic elements, including but not limited to an enhancer. The enhancer, which may enhance gene expression level, includes but is not limited to Tobacco Etch Virus (TEV) translation activator, CaMV35S enhancer and FMV35S enhancer.

Cry2Ab insecticidal protein and Cry 1 Ac insecticidal protein are two of many insecticidal proteins, which are insoluble parasporal crystal proteins produced by *Bacillus thuringiensis* (Abbr. as *Bt*)*.* Cry2Ab protein or Cry1Ac protein is ingested into the midgut by insects. The poisonous protein protoxin is dissolved in the alkaline pH environment of the insect midgut, and the N-and C-terminal of the protein are digested by alkaline proteases to convert the protoxin into active fragments. The active fragments bind to the receptors on the upper surface of the cell membrane of the insect midgut epithelium and insert into the intestinal membrane, resulting in the occurrence of focus of perforation in the cell membrane, destroying the osmotic pressure change inside and outside the cell membrane and the pH balance, etc., and disrupting the digestion process of the insect, eventually leading to its death.

The term "*Lepidoptera*"*,* including both moths and butterflies, is the largest order of pests in agriculture and forestry. It includes for example *Agrotis ypsilon* (Rottemberg), *Helicoverpa armigera* (Hubner), *Prodenia litura*, *Athetis lepigone,* and *Conogethes punctiferalis.*

The phosphinothricin N-acetyltransferase (PAT) gene can be an enzyme isolated from *Streptomyces viridochromogenes* strain, which catalyzes the conversion of L-phosphinothricin into its inactive form by acetylation so as to confer glufosinate ammonium herbicide tolerance to a plant. Phosphinothricin (PTC, 2-amino-4-methylphosphinyl-butyric acid) is an inhibitor of glutamine synthetase. PTC is a structural unit of the antibiotic 2-amino-4-methylphosphinyl-alanyl-alanine. This tripeptide (PTT) is active against Gram-positive and Gram-negative bacteria and fungus, *Botrytis cinerea.* The phosphinothricin N-acetyltransferase (PAT) gene may also serve as a selective marker gene.

The "glufosinate ammonium" (also known as phosphinothricin) refers to ammonium 2-amino-4-[hydroxy(methyl)phosphinyl]butyrate. Treatments with a "glufosinate ammonium herbicide" refer to treatments with any herbicide formulation containing glufosinate ammonium. It is within the skills of an ordinary agricultural technician to select application rates for a glufosinate ammonium formulation so as to realize a biologically effective amount. Treatment of a field containing plant materials from the transgenic soybean event DBN8205 with any herbicide formulation containing glufosinate ammonium will control the growth of weeds in the field and will not affect the growth or yield of the plant materials derived from the transgenic soybean event DBN8205.

The DNA construct is introduced into a plant via transformation methods including but not limited to *Agrobacterium*-mediated transformation, ballistic transformation and pollen tube pathway transformation.

The *agrobacterium-*mediated transformation is a commonly-used method for plant transformation. An exogenous DNA to be introduced into a plant is cloned into a vector between left and right border consensus sequences, i.e., a T-DNA region. The vector is transformed into *Agrobacterium* cells, which are subsequently used to infect plant tissues. The T-DNA region in the vector comprising the exogenous DNA is inserted into the plant genome.

Ballistic transformation is bombardment of plant cells with a vector comprising an exogenous DNA (particle-mediated biolistic transformation).

The pollen tube pathway transformation is a method for carrying an exogenous DNA into an embryo sac via a nucellus pathway using a natural pollen tube (also known as the transmitting tissue of pollen tube) formed after plant pollination.

After transformation, it is necessary to regenerate a transgenic plant from the transformed plant tissue, and select the progeny with the exogenous DNA using a suitable marker.

A DNA construct is an assembly of DNA molecules linked together that provides one or more expression cassettes. The DNA construct is preferably a plasmid that can self-replicate in a bacterial cell and contains various restriction endonuclease sites that are useful for introducing DNA molecules that provide functional genetic elements, i.e. promoters, introns, leader sequences, coding sequences, 3' terminator regions and other sequences. The expression cassettes contained in a DNA construct comprise genetic elements which are necessary for the transcription of a messenger RNA and can be designed to express in prokaryote cells or eukaryotic cells. Most preferably, the expression cassettes of the present invention are designed to express in plant cells.

A transgenic "event" is produced by transformation of plant cells with a heterologous DNA construct, comprising: inserting a nucleic acid expression cassette comprising at least one target gene into the genome of a plant through a transgenic method in order to generate a plant population, regenerating the plant population, and selecting a specific plant having the characteristics of insertion into a specific genome site. The term "event" refers to the original transformant and a progeny thereof that include a heterologous DNA. The term "event" also refers to a progeny produced by sexually crossing the original transformant with an individual of other varieties that include a heterologous DNA. Even after repeated back-crossing with a backcross parent, the inserted DNA and flanking genomic DNA from the original transformant parent are still present at the same chromosomal location in the crossing progeny. The term "event" also refers to a DNA sequence from the original transformant comprising an inserted DNA and flanking genomic sequences immediately adjacent to the inserted DNA, wherein the DNA sequence would be expected to be transferred to a progeny that is produced by sexually crossing a parental line that includes the inserted DNA (e.g., the original transformant and progeny thereof produced by selfing) with a parental line that does not contain the inserted DNA, and the progeny receives the inserted DNA including the target gene.

"Recombination" as used herein refers to a form of a DNA and/or a protein and/or an organism that normally could not be found in nature and as such is created by human intervention. Such human intervention may produce a recombinant DNA molecule and/or a recombinant plant. The "recombinant DNA molecule" is obtained by an artificial combination of two otherwise separated sequence segments, e.g., manipulation of isolated nucleic acid segments by chemical synthesis or by genetic engineering technology. The technology of manipulating nucleic acids is well known in the art.

The term "transgene" includes any cell, cell line, callus, tissue, plant part or plant, the genotype of which has been changed due to the presence of a heterologous nucleic acid. "Transgene" includes those transgenic organisms originally so altered as well as the individual progeny produced from the original transgenic organism by sexual cross or asexual propagation. The term "transgene" as used herein does not encompass the (chromosomal or extrachromosomal) alteration of genome by conventional plant breeding methods or by naturally occurring events, such as random cross-fertilization, non-recombinant viral infection, non-recombinant bacterial transformation, non-recombinant transposition or spontaneous mutation.

"Heterologous" as used herein refers to a first molecule which is not normally found in combination with a second molecule in nature. For example, a molecule may be derived from a first species and inserted into the genome of a second species. The molecule would thus be heterologous to the host and artificially introduced into the genome of the host cell.

A method for producing a transgenic soybean event DBN8205 having resistance to *Lepidoptera* insects and tolerance to glufosinate ammonium herbicide, comprises the following steps: firstly, sexually crossing a first parental soybean plant consisting of a soybean plant bred from the transgenic soybean event DBN8205 and a progeny thereof, with a second parental soybean plant that lacks resistance to *Lepidoptera* insects and tolerance to glufosinate ammonium herbicide, thereby producing a plurality of first progeny plants, wherein the transgenic soybean event DBN8205 and progeny thereof are obtained from the transformation using the expression cassette of the present invention that is resistant to *Lepidoptera* insects and tolerant to glufosinate ammonium herbicide; and then selecting a progeny plant that is resistant to the invasion of *Lepidoptera* insects and/or tolerant to glufosinate ammonium herbicide, thereby producing a soybean plant that is resistant to the invasion of *Lepidoptera* insects and tolerant to glufosinate ammonium herbicide. These steps can further include backcrossing the *Lepidoptera* insect-resistant and/or glufosinate ammonium-tolerant progeny plants with the second parental soybean plant or a third parental soybean plant, then screening the progeny by invasion of *Lepidoptera* insects, application of glufosinate ammonium herbicide or identification with molecular markers (e.g., the DNA molecule comprising the junctions identified from the 5' and 3' ends of the inserted sequence in the transgenic soybean event DBN8205) associated with the trait, thereby producing a *Lepidoptera* insect-resistant and glufosinate ammonium herbicide-tolerant soybean plant.

It is also to be understood that two different transgenic plants can also be crossed to produce a progeny that contains two independent and separately added exogenous genes. Selfing of an appropriate progeny can produce progeny plants that are homozygous for both of the added exogenous genes. Backcrossing with a parental plant and outcrossing with a non-transgenic plant as previously described are also contemplated, as is vegetative propagation.

The term "probe" means an isolated nucleic acid molecule to which a conventional detectable label or reporter molecule, for example, a radioactive isotope, a ligand, a chemiluminescent agent, or an enzyme is attached. Such a probe is complementary to a strand of a target nucleic acid, in the present invention, to a DNA strand from the genome of the transgenic soybean event DBN8205, no matter whether the genome DNA is derived from the transgenic soybean event DBN8205 or a seed or from a plant of the transgenic soybean event DBN8205 or a seed or extract thereof. Probes of the present invention include not only deoxyribonucleic acids or ribonucleic acids, but also polyamides and other probe materials that bind specifically to a target DNA sequence and can be used to detect the presence of that target DNA sequence.

The term "primer" is a fragment of isolated nucleic acid molecule which is annealed to a complementary target DNA strand by nucleic acid hybridization to form a hybrid between the primer and the target DNA strand, and then extends along the target DNA strand under the action of a polymerase (for example, a DNA polymerase). Primer pairs of the present invention refer to their use in the amplification of a target nucleic acid sequence, for example, by a polymerase chain reaction (PCR) or other conventional nucleic acid amplification methods.

Probes and primers are generally 11 polynucleotides or more, preferably 18 polynucleotides or more, more preferably 24 polynucleotides or more, most preferably 30 polynucleotides or more in length. Such probes and primers hybridize specifically to a target sequence under high stringency hybridization conditions. A probe that differs from the target DNA sequence and retains the ability to hybridize to the target DNA sequence may be designed by conventional methods. Nevertheless, preferably, probes and primers of the present invention have complete DNA sequence identity to consecutive nucleic acids in a target sequence.

Primers and probes based on the flanking genomic DNA and the inserted sequence of the present invention can be determined by conventional methods, for example, by isolating the corresponding DNA molecules from a plant material derived from the transgenic soybean event DBN8205, and determining the nucleic acid sequence of the DNA molecules. The DNA molecules comprise the transgenic inserted sequence and soybean genomic flanking sequences, and a fragment of the DNA molecule may be used as a primer or probe.

The nucleic acid probe and primer of the present invention hybridize to a target DNA sequence under stringent conditions. Any conventional nucleic acid hybridization or amplification method can be used to identify the presence of a DNA from the transgenic soybean event DBN8205 in a sample. Nucleic acid molecules or fragments thereof are capable of specifically hybridizing to other nucleic acid molecules under certain circumstances. As used herein, two nucleic acid molecules are capable of specifically hybridizing to each other if the two molecules are capable of forming an anti-parallel, double-stranded nucleic acid structure. A nucleic acid molecule is referred to be the "complement" of the other nucleic acid molecule if they exhibit complete complementarity. As used herein, two nucleic acid molecules are referred to exhibit "complete complementarity", if each nucleotide of a nucleic acid molecule is complementary to the corresponding nucleotide of the other nucleic acid molecule. Two nucleic acid molecules are referred to be "minimally complementary", if they can hybridize to each other with sufficient stability such that they can anneal and bind to each other under at least conventional "low stringency" conditions. Similarly, two nucleic acid molecules are referred to possess "complementarity", if they can hybridize to each other with sufficient stability such that they can anneal and bind to each other under conventional "high stringency" conditions. Departures from complete complementarity are permissible, as long as such departures do not completely preclude the two molecules from forming a double-stranded structure. To serve as a primer or probe, a nucleic acid molecule only needs to have sufficient complementarity in sequence such that it can form a stable double-stranded structure in a particular solvent and at particular salt concentrations employed.

As used herein, a substantially homologous sequence is a nucleic acid molecule which will specifically hybridize to a complementary strand of another matching nucleic acid molecule under high stringency conditions. Appropriate stringent conditions which promote DNA hybridization are known to those skilled in the art, for example, treating with 6.0 × sodium chloride/sodium citrate (SSC) at about 45°C, followed by washing with 2.0 × SSC at 50°C. For example, the salt concentration in the washing step can be about 2.0 × SSC at 50°C under low stringency conditions to about 0.2 × SSC at 50°C under high stringency conditions. In addition, the temperature in the washing step can be increased from room temperature (about 22°C) under low stringency conditions to about 65°C under high stringency conditions. Both temperature and salt concentration may be varied, or one of them may be held constant while the other variable is changed. Preferably, a nucleic acid molecule of the present invention can specifically hybridize to one or more nucleic acid molecules of SEQ ID NO: 1, SEQ ID NO: 2, SEQ ID NO: 3, SEQ ID NO: 4, SEQ ID NO: 5, SEQ ID NO: 6 and SEQ ID NO: 7, or complementary sequences thereof, or any fragment of the above sequences under moderate stringency conditions, for example at about 2.0 × SSC and about 65°C. More preferably, a nucleic acid molecule of the present invention can specifically hybridize to one or more nucleic acid molecules of SEQ ID NO: 1, SEQ ID NO: 2, SEQ ID NO: 3, SEQ ID NO: 4, SEQ ID NO: 5, SEQ ID NO: 6 and SEQ ID NO: 7, or complementary sequences thereof, or any fragment of the above sequences under high stringency conditions. A preferred marker nucleic acid molecule of the present invention comprises SEQ ID NO: 1, SEQ ID NO: 2, SEQ ID NO: 6 or SEQ ID NO: 7, or complementary sequences thereof, or any fragment of the above sequences. Another preferred marker nucleic acid molecule of the present invention has 80% to 100% or 90% to 100% sequence identity to SEQ ID NO: 1, SEQ ID NO: 2, SEQ ID NO: 6 or SEQ ID NO: 7, or complementary sequences thereof, or any fragment of the above sequences. SEQ ID NO: 1, SEQ ID NO: 2, SEQ ID NO: 6 and SEQ ID NO: 7 may be used as markers in plant breeding methods to identify a progeny of genetic cross. The hybridization of a probe to a target DNA molecule can be detected by any method well-known to those skilled in the art, including but not limited to, fluorescent labels, radioactive labels, antibody-based labels, and chemiluminescent labels.

Regarding the amplification of a target nucleic acid sequence using particular amplification primers (for example, PCR), "stringent conditions" are conditions that only permit a primer to hybridize to a target nucleic acid sequence in a DNA thermal amplification reaction, wherein the primer has a wild-type sequence corresponding to the target nucleic acid sequence (or its complementary sequence) and thus could bind to it, preferably to produce a unique amplification product, i.e. amplicon.

The term "specifically binding to (a target sequence)" means that under stringent hybridization conditions, a probe or primer hybridizes only to a target sequence in a sample comprising the target sequence.

As used herein, "amplicon" refers to a nucleic acid amplification product of a target nucleic acid sequence as a part of a nucleic acid template. For example, in order to determine whether a soybean plant is produced by sexually crossing the transgenic soybean event DBN8205 of the present invention, or whether a soybean sample collected from a field comprises the transgenic soybean event DBN8205, or whether a soybean extract (such as a meal, flour or oil) comprises the transgenic soybean event DBN8205, the DNA extracted from a soybean plant tissue sample or extract may be subject to a nucleic acid amplification method using a primer pair to produce an amplicon that is diagnostic for the presence of the DNA of the transgenic soybean event DBN8205. The primer pair includes a first primer derived from the flanking sequences adjacent to the insertion site of the inserted exogenous DNA in the plant genome, and a second primer derived from the inserted exogenous DNA. The amplicon with a certain length has a sequence that is also diagnostic for the transgenic soybean event DBN8205. The amplicon may have a length range that is the combined lengths of the primer pair plus one nucleotide base pair, preferably plus about 50 nucleotide base pairs, more preferably plus about 250 nucleotide base pairs, most preferably plus about 450 nucleotide base pairs or more.

Alternatively, a primer pair can be derived from flanking genomic sequences on both sides of the inserted DNA so as to produce an amplicon that includes the entire inserted nucleotide sequence. One of the primer pair derived from the plant genomic sequence may be located at a certain distance from the inserted DNA sequence, and this distance can range from one nucleotide base pair to about twenty thousand nucleotide base pairs. As used herein, the term "amplicon" specifically excludes primer dimers formed in a DNA thermal amplification reaction.

A nucleic acid amplification reaction can be accomplished by any one of various nucleic acid amplification methods known in the art, including polymerase chain reaction (PCR). A variety of nucleic acid amplification methods are known to those skilled in the art. PCR amplification methods have been developed to amplify up to 22 kb genomic DNA and up to 42 kb bacteriophage DNA. These methods as well as other DNA amplification methods known in the art can be used in the present invention. The inserted exogenous DNA sequence and flanking DNA sequences from the transgenic soybean event DBN8205 can be amplified on the genome of the transgenic soybean event DBN8205 using the primer sequences provided. After amplification, the PCR amplicon or cloned DNA is sequenced by standard sequencing methods.

DNA detection kits that are based on DNA amplification methods contain DNA molecules used as primers that hybridize specifically to a target DNA and amplify a diagnostic amplicon under appropriate reaction conditions. The kit may provide an agarose gel-based detection method or many known methods in the art for detecting a diagnostic amplicon. Provided by the present invention is a kit containing DNA primers homologous or complementary to any portion of the soybean genome in SEQ ID NO: 3 or SEQ ID NO: 4 and to any portion of the transgenic inserted region in SEQ ID NO: 5. A primer pair that is specifically identified as useful in a DNA amplification method is SEQ ID NO: 8 and SEQ ID NO: 9, which amplify a diagnostic amplicon homologous to a portion of 5' transgene/genome region of the transgenic soybean event DBN8205, wherein the amplicon comprises SEQ ID NO: 1. Other DNA molecules useful as DNA primers can be selected from SEQ ID NO: 5.

The amplicon produced by these methods may be detected by a plurality of techniques. One of such methods is Genetic Bit Analysis, in which a DNA oligonucleotide strand spanning the inserted DNA sequence and the adjacent flanking genomic DNA sequences is designed. The oligonucleotide strand is immobilized in wells of a microplate. Following the PCR amplification of the target region (using two primers respectively for the inside of the inserted sequence and the adjacent flanking genomic sequences), a single-stranded PCR product can be hybridized to the immobilized oligonucleotide strand and serve as a template for a single base extension reaction in which a DNA polymerase and specifically labeled ddNTPs for the next predictable base are used. The result can be obtained by fluorescent or ELISA-based methods. A signal represents the presence of the inserted/flanking sequence, which demonstrates that the amplification, hybridization, and single base extension are successful.

Another method is the Pyrosequencing technique. In this method, an oligonucleotide strand spanning the inserted DNA sequence and the adjacent genomic DNAjunction is designed. The oligonucleotide strand is hybridized to the single-stranded PCR product from the target region (using two primers respectively for the inside of the inserted sequence and the adjacent flanking genomic sequence) and incubated together with a DNA polymerase, ATP, sulfurylase, luciferase, apyrase, adenosine-5'-phosphosulfate and luciferin. The dNTPs are added separately and the produced light signal is measured. A light signal represents the presence of the inserted/flanking sequence, which demonstrates that the amplification, hybridization, and single or multi-base extension are successful.

Fluorescence polarization phenomenon as described by Chen et al. (Genome Res., 1999, 9: 492-498) is also a method for detecting the amplicon of the present invention. To use this method, it is necessary to design an oligonucleotide strand spanning the inserted DNA sequence and the adjacent genomic DNAjunction. The oligonucleotide strand is hybridized to the single-stranded PCR product from the target region (using two primers respectively for the inside of the inserted sequence and the adjacent flanking genomic sequences) and incubated together with a DNA polymerase and a fluorescently-labeled ddNTP. Single base extension results in the incorporation of ddNTP. Such incorporation can be measured as a change in polarization using a fluorometer. A change in polarization represents the presence of the inserted/flanking sequence, which demonstrates that the amplification, hybridization, and single base extension are successful.

Taqman is described as a method for detecting and quantitatively analyzing the presence of a DNA sequence and is fully described in the instructions provided by the manufacturer. Now, it is briefly illustrated as follows. A FRET oligonucleotide probe spanning the inserted DNA sequence and the adjacent genomic flanking junction is designed. The FRET probe and PCR primers (using two primers respectively for the inside of the inserted sequence and the adjacent flanking genomic sequences) are subject to reaction cycles in the presence of a thermostable polymerase and dNTPs. Hybridization of the FRET probe results in cleavage between the fluorescent moiety and quenching moiety on the FRET probe and release of the fluorescent moiety. The generation of a fluorescent signal represents the presence of the inserted/flanking sequence, which demonstrates that the amplification and hybridization are successful.

Based on the principle of hybridization, suitable techniques for detecting plant materials from the transgenic soybean event DBN8205 also comprise Southern blot, Northern blot and in situ hybridization. Particularly, the suitable techniques involve incubating a probe with a sample, washing them to remove unbound probe, and detecting whether the probe has been hybridized. The detection method is dependent on the type of the label attached to the probe, for example, a radioactively-labeled probe can be detected by exposure and development of X-ray film, or an enzymatically-labeled probe can be detected by conversion of a substrate to effect a color change.

The application of molecular markers in sequence detection has been described by Tyangi et al. (Nature Biotech., 1996, 14: 303-308), which is briefly described as follows. A FRET oligonucleotide probe spanning the inserted DNA sequence and the adjacent genomic flanking junctions is designed. Due to the unique structure of the FRET probe, it contains a secondary structure that keeps the fluorescent moiety and the quenching moiety in close proximity. The FRET probe and PCR primers (using two primers respectively for the inside of the inserted sequence and the flanking genomic sequences) are subject to reaction cycles in the presence of a thermostable polymerase and dNTPs. Following successful PCR amplification, hybridization of the FRET probe to the target sequence results in loss of the probe's secondary structure, so that the fluorescent moiety and the quenching moiety are spatially separated and a fluorescent signal is generated. The generation of a fluorescent signal represents the presence of the inserted/flanking sequence, which demonstrates that the amplification and hybridization are successful.

Other described methods, such as microfluidics, provide methods and devices for isolating and amplifying DNA samples. Optical dyes are used to detect and determine specific DNA molecules. Nanotube devices, which comprise an electronic sensor for detecting DNA molecules or nanobeads for binding specific DNA molecules and thus can be detected, are useful for detecting the DNA molecules of the present invention.

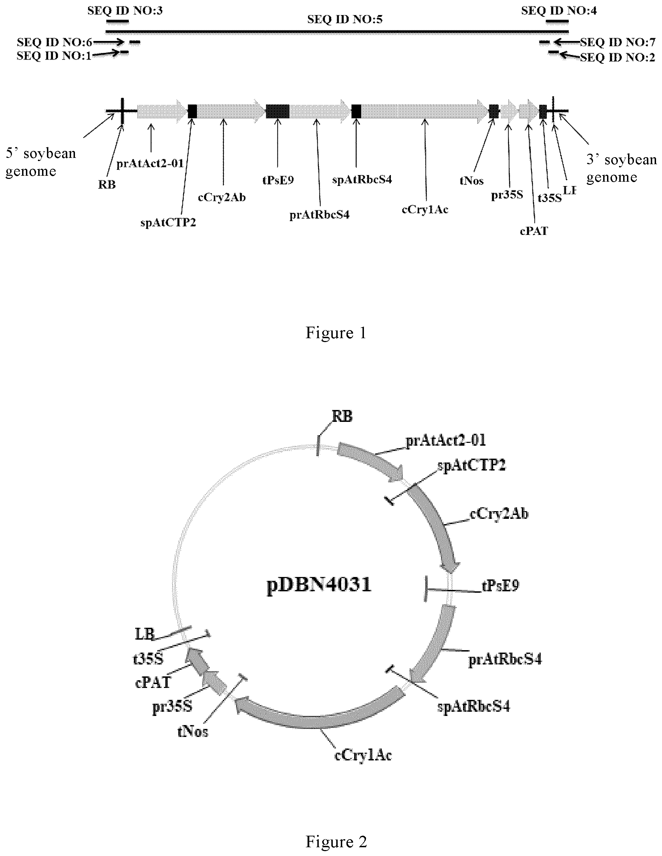

DNA detection kits can be developed using the compositions described herein and methods described or known in the field of DNA detection. The kits are useful for identifying whether a sample contains DNA of the transgenic soybean event DBN8205 and can also be applied to breed soybean plants containing the DNA of the transgenic soybean event DBN8205. The kits may comprise DNA primers or probes homologous or complementary to at least a portion of SEQ ID NO: 1, 2, 3, 4 or 5, or comprise other DNA primers or probes homologous or complementary to the DNA contained in the transgenic genetic elements of DNA. These DNA sequences can be used in DNA amplification reactions or as probes in DNA hybridization methods. The DNA structure of the transgenic inserted sequence and the soybean genomic junctions contained in the soybean genome and illustrated in Figure 1 and Table 1 comprises: the soybean plant DBN8205 flanking genomic region adjacent to the 5' end of the transgenic inserted sequence; a portion of an inserted sequence from the right border region (RB) of *Agrobacterium;* a first expression cassette consisting of an Arabidopsis ACT2 promoter (prAtAct2-01), operably linked to the Arabidopsis chloroplast transit peptide gene (spAtCTP2), further operably linked to the insect-resistant cCry2Ab gene of *Bacillus thuringiensis,* further operably linked to a pea (*Pisum sativu*) RbcS gene terminator (tPsE9); a second expression cassette consisting of an Arabidopsis ribulose-1,5-bisphosphate carboxylase small subunit gene promoter (prAtRbcS4), operably linked to an Arabidopsis ribulose-1,5-bisphosphate carboxylase small subunit gene chloroplast transit peptide gene (spAtRbcS4), further operably linked to the insect-resistant cCry1Ac gene of *Bacillus thuringiensis,* further operably linked to a nopaline synthetase terminator (tNos); a third expression cassette consisting of a cauliflower mosaic virus 35S promoter (pr35S), operably linked to a glufosinate ammonium-tolerant phosphinothricin-N-acetyltransferase gene (cPAT) of *Streptomyces,* further operably linked to a cauliflower mosaic virus 35S terminator (t35S); a portion of an inserted sequence from the left border region (LB) of *Agrobacterium;* and soybean plant DBN8205 flanking genomic region at the 3' end of the transgenic inserted sequence (SEQ ID NO: 5). In DNA amplification methods, the DNA molecules useful as primers can be any portion derived from the transgenic inserted sequence in the transgenic soybean event DBN8205, or any portion derived from the flanking soybean genomic DNA sequence in the transgenic soybean event DBN8205.

The transgenic soybean event DBN8205 can be used in combination with other transgenic soybean varieties, for example, herbicide (such as glyphosate and dicamba)-tolerant transgenic soybean varieties, or transgenic soybean varieties carrying other insect-resistant genes. Various combinations of these different transgenic events, when used together with the transgenic soybean event DBN8205 of the present invention for breeding, can provide improved hybrid transgenic soybean varieties resistant to various insects and tolerant to various herbicides. These varieties can exhibit better performances, as compared with non-transgenic varieties and single-trait transgenic varieties.

For example, the invention provides the superimposed transgenic soybean event DBN8205 × DBN8002 × DBN9004 obtained by crossing DBN8205, DBN8002 and DBN9004. The superimposed transgenic soybean event DBN8205 × DBN8002 × DBN9004 comprises a cCry2Ab gene, a cCry1Ac gene, a cPAT gene, a cVip3Aa gene, and a cEPSPS gene inserted into specific sites within the genome of a soybean cell to effectively control major *Lepidoptera* pests of soybean in South America (Argentina and Brazil) and China by three insect-resistance mechanisms; also, the cPAT gene and the cEPSPS gene in the superimposed transgenic soybean event can confer tolerance to glufosinate ammonium herbicide and glyphosate herbicide to soybean plants without compromising the yield.

The term "superimposed" means that at least two transgenic events having desired traits are combined into the same plant. The transgenic events are superimposed by crossing between parents of the transgenic event having the desired traits and then identifying a progeny having all of these desired traits. Superposition of transgenic events can be used to combine two or more different traits, including, for example, two or more different insect resistance traits, two or more herbicide resistance traits, and/or insect resistance traits and herbicide resistance traits.

The superimposed transgenic soybean event DBN8205 × DBN8002 × DBN9004 according to the present invention is also referred to as a soybean plant DBN8205 × DBN8002 × DBN9004, including plants and seeds of the superimposed transgenic soybean event DBN8205 × DBN8002 × DBN9004, together with plant cells or regenerable parts thereof, wherein the plant parts of the superimposed transgenic soybean event DBN8205 × DBN8002 × DBN9004 include but are not limited to cells, pollens, ovules, flowers, buds, roots, stems, leaves, pods and products derived from superimposed transgenic soybean event DBN8205 × DBN8002 × DBN9004 such as soybean cakes, powders and oils, specifically lecithin, fatty acids, glycerol, sterols, edible oils, defatted soy flakes, including defatted and baked soy flours, soy milk clots, bean curd, soy protein concentrates, isolated soy proteins, hydrolyzed vegetable proteins, organized soy proteins and soy protein fibers.

The transgenic soybean event DBN8205 of the present invention is resistant to the ingestion damage caused by *Lepidoptera* pests, and tolerant to the phytotoxicity of agricultural herbicides containing glufosinate ammonium. The dual-trait soybean plant expresses the Cry2Ab protein and Cry1Ac protein of *Bacillus thuringiensis,* providing resistance to the ingestion damage caused by *Lepidoptera* pests, and expresses glufosinate ammonium-resistant phosphinothricin-N-acetyl-transferase (PAT) protein of *Streptomyces,* conferring glufosinate ammonium tolerance to the plant. The dual-trait soybean has the following advantages: 1) economic losses due to *Lepidoptera* pests (such as *Helicoverpa armigera* (Hubner), *Spodoptera litura*, *Spodoptera exigua* and *Agrotis ypsilon* (Rottemberg)) are avoided, wherein *Helicoverpa armigera* (Hubner), *Spodoptera litura*, *Spodoptera exigua* and *Agrotis ypsilon* (Rottemberg) are major pests in soybean planting areas; 2) application of agricultural herbicides containing glufosinate ammonium confers the soybean an ability of broad-spectrum control of weeds; and 3) soybean production is not reduced. In addition, the transgenes encoding insect-resistant and glufosinate ammonium-tolerant traits are linked on the same DNA segment, and exist in a single locus of the transgenic soybean event DBN8205 genome, which provide an enhanced breeding efficacy and allow to track the transgenic inserted fragments in the propagating population and progeny thereof by using molecular markers. Meanwhile, in the detection methods of the present invention, SEQ ID NO: 1 or a complementary sequence thereof, SEQ ID NO: 2 or a complementary sequence thereof, SEQ ID NO: 6 or a complementary sequence thereof, or SEQ ID NO: 7 or a complementary sequence thereof can be used as DNA primers or probes to produce amplification products diagnostic for the transgenic soybean event DBN8205 or progeny thereof, and can rapidly, accurately and stably identify the presence of plant materials derived from the transgenic soybean event DBN8205.

### BRIEF DESCRIPTION OF SEQUENCES

SEQ ID NO: 1 a sequence of 22 nucleotides in length that is adjacent to the insertion junction at the 5' end of the inserted sequence in the transgenic soybean event DBN8205, wherein nucleotides 1-11 and nucleotides 12-22 are located respectively at each side of the insertion site in the soybean genome;
SEQ ID NO: 2 a sequence of 22 nucleotides in length that is adjacent to the insertion junction at the 3' end of the inserted sequence in the transgenic soybean event DBN8205, wherein nucleotides 1-11 and nucleotides 12-22 are located respectively at each side of the insertion site in the soybean genome;
SEQ ID NO: 3 a sequence of 634 nucleotides in length that is adjacent to the insertion junction at the 5' end of the inserted sequence in the transgenic soybean event DBN8205;
SEQ ID NO: 4 a sequence of 642 nucleotides in length that is adjacent to the insertion junction at the 3' end of the inserted sequence in the transgenic soybean event DBN8205;
SEQ ID NO: 5 the entire T-DNA sequence, 5' and 3' end soybean genomic flanking sequences;
SEQ ID NO: 6 spans the DNA sequence of the pDBN4031 construct and a sequence of prAtAct2-01 transcription origin;
SEQ ID NO: 7 spans a t35S transcriptional terminator sequence and the DNA sequence of the pDBN4031 construct;
SEQ ID NO: 8 a first primer for amplifying SEQ ID NO: 3;
SEQ ID NO: 9 a second primer for amplifying SEQ ID NO: 3;
SEQ ID NO: 10 a first primer for amplifying SEQ ID NO: 4;
SEQ ID NO: 11 a second primer for amplifying SEQ ID NO: 4;
SEQ ID NO: 12 a primer on 5' flanking genomic sequence;
SEQ ID NO: 13 a primer on T-DNA, which is paired with SEQ ID NO: 12;
SEQ ID NO: 14 a primer on 3' flanking genomic sequence, which can be used in pair with SEQ ID NO: 12 to detect whether a transgene is homozygous or heterozygous;
SEQ ID NO: 15 a primer on T-DNA, which is paired with SEQ ID NO: 14;
SEQ ID NO: 16 a first primer for detecting cCry2Ab gene in Taqman;
SEQ ID NO: 17 a second primer for detecting cCry2Ab gene in Taqman;
SEQ ID NO: 18 a probe for detecting cCry2Ab gene in Taqman;
SEQ ID NO: 19 a first primer for detecting cCry1Ac gene in Taqman;
SEQ ID NO: 20 a second primer for detecting cCry1Ac gene in Taqman;
SEQ ID NO: 21 a probe for detecting cCry1Ac gene in Taqman;
SEQ ID NO: 22 a first primer for detecting cPAT gene in Taqman;
SEQ ID NO: 23 a second primer for detecting cPAT gene in Taqman;
SEQ ID NO: 24 a probe for detecting cPAT gene in Taqman;
SEQ ID NO: 25 a first primer for soybean endogenous gene lectin;
SEQ ID NO: 26 a second primer for soybean endogenous gene lectin;
SEQ ID NO: 27 a probe for cCry2Ab gene in Southern blot assay;
SEQ ID NO: 28 a probe for cCry1Ac gene in Southern blot assay;
SEQ ID NO: 29 a probe for cPAT gene in Southern blot assay;
SEQ ID NO: 30 a primer on T-DNA, in the same direction as SEQ ID NO: 13;
SEQ ID NO: 31 a primer on T-DNA, in the same direction as SEQ ID NO: 15;
SEQ ID NO: 32 a primer on T-DNA, in an opposite direction to SEQ ID NO: 13;
SEQ ID NO: 33 a primer on T-DNA, in an opposite direction to SEQ ID NO: 13;
SEQ ID NO: 34 a primer on T-DNA, in an opposite direction to SEQ ID NO: 15;
SEQ ID NO: 35 a primer on T-DNA, in an opposite direction to SEQ ID NO: 15;
SEQ ID NO:36 a nucleotide sequence of prAtAct2-02 on the recombinant expression vector pDBN4032;
SEQ ID NO:37 a nucleotide sequence of tOsMth on the recombinant expression vector pDBN4032;
SEQ ID NO:38 a nucleotide sequence of tMtPt1 on the recombinant expression vector pDBN4032.

The technical solutions of the present invention will be further described below in detail with reference to the accompanying figures and examples.

### BRIEF DESCRIPTION OF THE FIGURES

Figure 1 is a structural scheme of the junction sites between the transgenic inserted sequences and the soybean genome, and a scheme of relative positions of nucleic acid sequences for detecting soybean plant DBN8205 (for the scheme of relative positions, please refer to Wm82.a4 RefGen) in the nucleic acid sequences for detecting soybean plants DBN8205 and detection methods thereof according to the present invention;
Figure 2 is a structural scheme of the recombinant expression vector pDBN4031 in the nucleic acid sequences for detecting the soybean plant DBN8205 and detection methods thereof according to the present invention;
Figure 3 is a structural scheme of the recombinant expression vector pDBN4032 according to the present invention;
Figure 4 shows the field effect of the transgenic soybean event DBN8205 under the conditions of naturally occurring *Helicoverpa armigera;*
Figure 5 shows the field effect of the transgenic soybean event DBN8205 under the conditions of naturally occurring *Spodoptera exigua;*
Figure 6 shows the field effect of the transgenic soybean event DBN8205 under the conditions of naturally occurring *Argyrogramma agnata*;
Figure 7 shows the field effect in area one of the transgenic soybean event DBN8205 under the conditions of naturally occurring *Leguminivora glycinivorella.*

### PARTICULAR EMBODIMENTS OF THE INVENTION

Technical solutions of the nucleic acid sequences for detecting the soybean plant DBN8205 and detection methods thereof according to the present invention will be further illustrated below with reference to the specific examples.

### Example 1: Cloning and transformation

### 1.1 Vector cloning

A recombinant expression vector pDBN4031 (as shown in Figure 2) was constructed by using standard gene cloning techniques. The vector pDBN4031 comprises three transgenic expression cassettes arranged in tandem: the first expression cassette consisting of an Arabidopsis ACT2 promoter (prAtAct2-01), operably linked to the Arabidopsis chloroplast transit peptide gene (spAtCTP2), further operably linked to the insect-resistant cCry2Ab gene of *Bacillus thuringiensis,* further operably linked to a pea RbcS gene terminator (tPsE9); the second expression cassette consisting of an Arabidopsis ribulose-1,5-bisphosphate carboxylase small subunit gene promoter (prAtRbcS4), operably linked to an Arabidopsis ribulose-1,5-bisphosphate carboxylase small subunit gene chloroplast transit peptide gene (spAtRbcS4), further operably linked to the insect-resistant cCry1Ac gene of *Bacillus thuringiensis,* further operably linked to a nopaline synthetase terminator (tNos); the third expression cassette consisting of a cauliflower mosaic virus 35S promoter (pr35S), operably linked to a glufosinate ammonium-tolerant phosphinothricin-N-acetyltransferase gene (cPAT) of *Streptomyces,* further operably linked to a cauliflower mosaic virus 35S terminator (t35S).

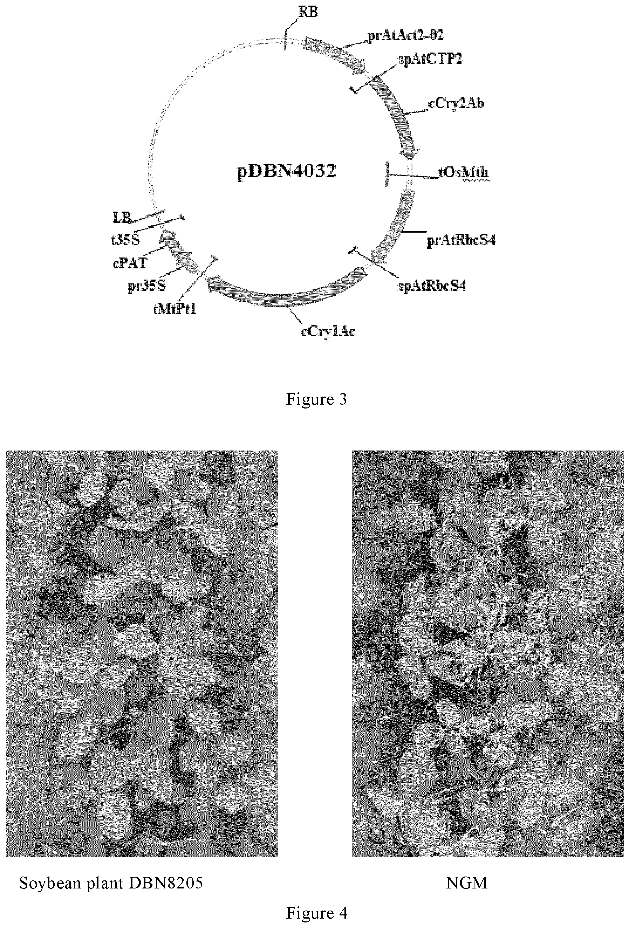

A recombinant expression vector pDBN4032 (as shown in Figure 3) was constructed by using standard gene cloning techniques. The vector pDBN4032 comprises three transgenic expression cassettes arranged in tandem: the first expression cassette consisting of an Arabidopsis ACT2 promoter (prAtAct2-02) (SEQ ID NO:36), operably linked to the Arabidopsis chloroplast transit peptide gene (spAtCTP2), further operably linked to the insect-resistant cCry2Ab gene of *Bacillus thuringiensis,* further operably linked to a metallothionein-like protein gene terminator (tOsMth) (SEQ ID NO:37); the second expression cassette consisting of an Arabidopsis ribulose-1,5-bisphosphate carboxylase small subunit gene promoter (prAtRbcS4), operably linked to an Arabidopsis ribulose-1,5-bisphosphate carboxylase small subunit gene chloroplast transit peptide gene (spAtRbcS4), further operably linked to the insect-resistant cCry1Ac gene of *Bacillus thuringiensis,* further operably linked to a *Medicago truncatula* phosphate transporter 1 gene terminator (tMtPt1) (SEQ ID NO:38); the third expression cassette consisting of a cauliflower mosaic virus 35S promoter (pr35S), operably linked to a glufosinate ammonium-tolerant phosphinothricin-N-acetyltransferase gene (cPAT) of *Streptomyces,* further operably linked to a cauliflower mosaic virus 35S terminator (t3 5 S).

*Agrobacterium* LBA4404 (Invitrogen, Chicago, USA; Cat. No: 18313-015) was transformed with the vector pDBN4031 and pDBN4032 respectively by liquid nitrogen method, and 4-[hydroxy(methyl)phosphinyl]-DL-homoalanine was used as a selective marker for screening transformed cells.

### 1.2 Plant transformation

Transformation was performed by a conventional *Agrobacterium*-mediated transformation method, comprising: co-culturing the sterile-cultured soybean cotyledonary node tissues with *Agrobacterium* containing the vector pDBN4031 as described in Example 1.1 to transfer the T-DNA in the recombinant expression vector pDBN4031 into the soybean chromosomes, so as to produce the transgenic soybean event containing the recombinant expression vector pDBN4031.

As described above, co-culturing the sterile-cultured soybean cotyledonary node tissues with *Agrobacterium* containing vector pDBN4032 as described in Example 1.1 to transfer the T-DNA in the recombinant expression vector pDBN4032 into the soybean chromosomes, so as to produce the transgenic soybean event containing the recombinant expression vector pDBN4032.

Briefly, the *Agrobacterium*-mediated soybean transformation comprises: germinating mature soybean seeds (soybean variety Jack) in a soybean germination medium (3.1 g/L B5 salt, B5 vitamin, 20 g/L sucrose, and 8 g/L agar; pH 5.6); inoculating the seeds onto the germination medium, and culturing them under the conditions of: a temperature of 25 ± 1°C and a photoperiod (light/dark) of 16 h/8 h; at 4-6 days after germination, collecting fresh green soybean sterile plantlets with inflated cotyledonary node; excising the hypocotyls approximately 3-4 mm below the cotyledonary node; making longitudinal cuts through the cotyledons, and removing apical buds, lateral buds and seminal roots; wounding at the cotyledonary node with the back of a surgical blade, and contacting the wounded cotyledonary node tissues with an *Agrobacterium* suspension, wherein *Agrobacterium* can deliver the nucleotide sequence of cCry2Ab gene, the nucleotide sequence of cCry1Ac gene and the nucleotide sequence of cPAT gene in pDBN4031 (or the nucleotide sequence of cCry2Ab gene, the nucleotide sequence of cCry1Ac gene and the nucleotide sequence of cPAT gene in pDBN4032) to the wounded cotyledonary node tissues (step 1: infection step). In this step, the cotyledonary node tissues were preferably immersed in the *Agrobacterium* suspension (OD₆₆₀=0.5-0.8, infection medium (2.15 g/L MS salt, B5 vitamin, 20 g/L sucrose, 10 g/L glucose, 40 mg/L acetosyringone (AS), 4 g/L 2-morpholine ethanesulfonic acid (MES), and 2 mg/L zeatin (ZT); pH 5.3) to start the infection. The cotyledonary node tissues were co-cultured with *Agrobacterium* for a period of time (3 days) (step 2: co-culture step). Preferably, the cotyledonary node tissues were cultured in a solid medium (containing 4.3 g/L MS salt, B5 vitamin, 20 g/L sucrose, 10 g/L glucose, 4 g/L MES, 2 mg/L ZT, and 8 g/L agar; pH 5.6) after the infection step. After the co-culture step, there may be an optional "recovery" step. In the "recovery" step, the recovery medium (3.1 g/L B5 salt, B5 vitamin, 1 g/L MES, 30 g/L sucrose, 2 mg/L ZT, 8 g/L of agar, 150 mg/L cephalosporin, 100 mg/L glutamic acid, and 100 mg/L aspartic acid; pH 5.6) comprises at least one antibiotic (150-250 mg/L cephalosporin) known to inhibit the growth of *Agrobacterium,* without addition of any selective agent for plant transformants (step 3: recovery step). Preferably, the tissue blocks regenerated from the cotyledonary node were cultured in a solid medium comprising an antibiotic but no selective agent to eliminate *Agrobacterium* and provide a recovery stage for the infected cells. Subsequently, the tissue blocks regenerated from the cotyledonary node were cultured on a medium containing a selective agent (4-[(hydroxy(methyl)phosphinyl)]-DL-homoalanine), and the growing transformed calli were selected (step 4: selection step). Preferably, the tissue blocks regenerated from the cotyledonary node were cultured in a screening solid selective medium (3.1 g/L B5 salt, B5 vitamin, 1 g/L MES, 30 g/L sucrose, 1 mg/L 6-benzyladenine (6-BAP), 8 g/L agar, 150 mg/L cephalosporin, 100 mg/L glutamic acid, 100 mg/L aspartic acid, and 10 mg/L 4-(hydroxy(methyl)phosphinyl)-DL-homoalanine; pH 5.6) comprising a selective agent, which resulted in the continuous growth of the transformed cells. Then, the transformed cells regenerated into plants (step 5: regeneration step). Preferably, the tissue blocks regenerated from the cotyledonary node growing on the medium containing a selective agent were cultured on a solid medium (B5 differentiation medium and B5 rooting medium) to regenerate plants.

The resistant tissues obtained from screening were transferred to B5 differentiation medium (3.1 g/L B5 salt, B5 vitamin, 1 g/L MES, 30 g/L sucrose, 1 mg/L ZT, 8 g/L agar, 150 mg/L cephalosporin, 50 mg/L glutamic acid, 50 mg/L aspartic acid, 1 mg/L gibberellin, 1 mg/L auxin, and 5 mg/L 4-(hydroxy(methyl)phosphinyl)-DL-homoalanine; pH 5.6), and cultured for differentiation at 25°C. The differentiated plantlets were transferred to B5 rooting medium (3.1 g/L B5 salt, B5 vitamin, 1 g/L MES, 30 g/L sucrose, 8 g/L agar, 150 mg/L cephalosporin, and 1 mg/L indole-3-butyric acid (IBA)), and cultured at 25°C. When the plantlets reached about 10 cm in height, they were moved to greenhouse and cultured until fruiting. In the greenhouse, they were cultured at 26°C for 16 hours and then at 20°C for 8 hours per day.

### 1.3. Identification and screening of transgenic events

A total of 1037 independent transgenic T₀ plants were produced by the vector pDBN4031. In order to screen out a transgenic event with optimum performance, the above 1037 independent transgenic T₀ single plants were moved into the greenhouse for transplantation, cultivation and propagation to afford transgenic T₁ single plants.

Since the genetic transformation process of soybean using mature soybean seeds and glufosinate ammonium as a selective agent tends to generate false-positive transgenic events, the T₁ generation was sprayed with glufosinate ammonium to identify positive transgenic events, and a total of 137 positive transgenic single plants were obtained. By TaqMan^{™} analysis, the above 137 transgenic soybean plants were detected for the presence of single-copied cCry2Ab gene, cCry1Ac gene and cPAT gene and the absence of backbone sequence of the vector; and a total of 84 transgenic single plants were obtained. By analysis of the transgenic insertion sites, a total of 30 transgenic single plants were obtained by screening, in which the sequences on both sides of T-DNA were intact, the T-DNA was not inserted into important genes of the soybean genome and the gene insertion did not result in any large open reading frame (ORF). By evaluation and comparison of the resistance against major target insects (such as *Helicoverpa armigera* (Hubner), *Prodenia litura*, *Spodoptera exigua,* and *Agrotis ypsilon*), a total of 25 transgenic single plants with good insect resistance were obtained by screening. Since genetic transformation and gene insertion may affect agronomic traits of soybean plants (such as seedling vigor, propagation period, plant height or lodging), the above 25 transgenic T₂-generation single plants were planted in a field to identify the agronomic trait performance of the transgenic T₂ single plants at different stages (seedling stage to full flowering stage, initial grain-forming stage to maturity stage); then, by means of selfing and backcross breeding, it is screened whether the agronomic traits, molecular biology, resistance against the target insects and glufosinate ammonium tolerance of the transgenic soybean plants can be stably inherited under the conditions of different generations, different geographical environments and/or different background materials, and three excellent transgenic soybean events DBN8205, pDBN4031-1 and pDBN4031-2 were selected, wherein the transgenic soybean event DBN8205 has the most excellent traits (see Examples 6 to 9).

According to the method of screening excellent transgenic soybean events DBN8205, pDBN4031-1 and pDBN4031-2 for the vector pDBN4031 described above, three excellent transgenic soybean events pDBN4032-1, pDBN4032-2 and pDBN4032-3 were selected for the constructed recombinant expression vector pDBN4032, all of which had single-copy transgenes.

By comparing the evaluation of resistance (see Example 6) against major target insects (*Spodoptera litura* and *Spodoptera exigua*) of transgenic soybean events DBN8205, pDBN4031-1 and pDBN4031-2 selected by vector pDBN4031 and transgenic soybean events pDBN4032-1, pDBN4032-2 and pDBN4032-3 selected by vector pDBN4032, it is indicated that the design of vector pDBN4031 is more excellent, which is an excellent vector obtained by sufficiently considering and analyzing combinations and interactions of regulatory elements; meanwhile, it is indicated that the resistance of transgenic soybean event DBN8205 against major target insects (*Spodoptera litura* and *Spodoptera exigua*) is the most excellent.

### Example 2: Detection of the transgenic soybean event DBN8205 by TaqMan

Using a plant DNA extraction kit (DNeasy Plant Maxi Kit, Qiagen), the genomic DNA was extracted from the leaf (about 100 mg) of the transgenic soybean event DBN8205 as a sample, and the copy numbers of cCry2Ab gene, cCry1Ac gene and cPAT gene were detected by a fluorescent quantitative PCR method using Taqman probe. Meanwhile, a wild-type soybean plant as a control was subject to the detection and analysis according to the above methods. Experiments were carried out in triplicate and the average value was calculated.

The specific method is as follows:
Step 1: 100 mg leaf of the transgenic soybean event DBN8205 was ground to a homogenate in a mortar with liquid nitrogen, and each sample was prepared in triplicate;
Step 2: the genomic DNAs of the above samples were extracted using a plant DNA extraction kit (DNeasy Plant Maxi Kit, Qiagen); for detailed methods, please refer to the Product Instructions);
Step 3: the concentrations of the genomic DNAs of the above samples were measured using ultra micro-spectrophotometer (NanoDrop 2000, Thermo Scientific);
Step 4: the concentrations of the genomic DNAs of the above samples were adjusted to the same concentration value ranging from 80 to 100 ng/µL;
Step 5: the copy numbers of the samples were identified by a fluorescent quantitative PCR method using Taqman probe, wherein the identified sample with a known copy number was used as a standard and a sample from a wild-type soybean plant was used as control. Each sample was tested in triplicate and the average value was calculated. The sequences of primers and probes for fluorescent quantitative PCR are as follows:

The following primers and probe are used for detecting the sequence of cCry2Ab gene:
Primer 1: gtccacgagaatggatcaatga as set forth in SEQ ID NO: 16 in SEQUENCE LISTING;
Primer 2: gtgtggcgtgaataggtgaaatag as set forth in SEQ ID NO: 17 in SEQUENCE LISTING;
Probe 1: ctggctcccaacgactataccgggttt as set forth in SEQ ID NO: 18 in SEQUENCE LISTING;

The following primers and probe are used for detecting the sequence of cCry1Ac gene:
Primer 3: gacacagtttctgctcagcgag as set forth in SEQ ID NO: 19 in SEQUENCE LISTING;
Primer 4: cccagatgatgtcaactagtccg as set forth in SEQ ID NO: 20 in SEQUENCE LISTING;
Probe 2: cgtgccaggtgctgggttcgttc as set forth in SEQ ID NO: 21 in SEQUENCE LISTING.

The following primers and probe are used for detecting the sequence of cPAT gene:
Primer 5: gagggtgttgtggctggtattg as set forth in SEQ ID NO: 22 in SEQUENCE LISTING;
Primer 6: tctcaactgtccaatcgtaagcg as set forth in SEQ ID NO: 23 in SEQUENCE LISTING;
Probe 3: cttacgctgggccctggaaggctag as set forth in SEQ ID NO: 24 in SEQUENCE LISTING.

The PCR reaction system comprises:

| | |
|---|---|
| JumpStart^{™} Taq ReadyMix^{™} (Sigma) | 10 µL |
| 50 × primer/probe mix | 1 µL |
| Genomic DNA | 3 µL |
| Double distilled water (ddH₂O) | 6 µL |

wherein the 50 × primer/probe mix comprises 45 µl of each primer at a concentration of 1 mM, 50 µl of a probe at a concentration of 100 µM, and 860 µl of 1 × TE buffer (10 mM Tris-HCl, 1 mM EDTA; pH 8.0), and was stored in an amber tube at 4°C.

PCR reaction condition was set as follows:

| Step | Temperature | Time |
|---|---|---|
| 1 | 95°C | 5 min |
| 2 | 95°C | 30 s |
| 3 | 60°C | 1 min |
| 4 | go back to step 2 and repeat 40 times | |

Data was analyzed using fast real-time fluorescent quantitative PCR system software (Applied Biosystems 7900HT Fast Real-Time PCR System SDS v2.3, Applied Biosystems). The results demonstrate that the resultant transgenic soybean event DBN8205 is a single copy.

### Example 3: Analysis of the insertion site of the transgenic soybean event DBN8205

### 3.1 Extraction of genomic DNA

DNA was extracted by the conventional CTAB (cetyl trimethyl ammonium bromide) method: after 2 g young leaf of the transgenic soybean event DBN8205 was ground to powder in liquid nitrogen, 0.5 mL CTAB DNA extraction buffer (20 g/L CTAB, 1.4 M NaCl, 100 mM Tris-HCl, and 20 mM EDTA (edetic acid), with a pH adjusted to pH 8.0 with NaOH) preheated at 65°C was added, then they were mixed thoroughly and extracted at 65°C for 90 min; 0.5 volume of phenol and 0.5 volume of chloroform were added and mixed by inversion; the mixture was centrifuged at 12,000 rpm (revolution per minute) for 10 min; the supernatant was pipetted and 2 volumes of absolute ethanol was added; the centrifuge tube was gently shaken and then left standing at 4°C for 30 min; the centrifuge tube was centrifuged again at 12,000 rpm for 10 min such that DNA was collected to the bottom of the tube; the supernatant was discarded, and the pellet was washed with 1 mL of 70% (mass concentration) ethanol; the mixture was centrifuged at 12,000 rpm for 5 min; the pellet was dried in vacuum or blown dry on a super clean bench; and the resultant DNA pellet was dissolved in an appropriate amount of TE buffer and stored at -20°C.

### 3.2 Analysis of flanking DNA sequences

The concentration of the above extracted DNA sample was measured. The concentration of the sample to be tested is 80-100 ng/µL. The genomic DNA was digested using restriction endonucleases *EcoR* I (5' end analysis) and *EcoR* V (3' end analysis) respectively. Each enzymatic digestion system was added with 26.5 µL of genomic DNA, 0.5 µL of the above restriction endonucleases and 3 µL of enzymatic digestion buffer (all employed restriction endonucleases are enzymes with assorted buffers thereof or generic buffers from NEB company (now known as NEBCutSmart)), and was digested for 1 hour. After digestion, the enzymatic digestion system was added with 70 µL of absolute ethanol, kept in ice bath for 30 min, and centrifuged at 12,000 rpm for 7 min. After discarding the supernatant, the pellet was blow-dried, then added with 8.5 µL of double distilled water, 1 µL of 10 × T₄-DNA ligase buffer (NEB T4 DNA Ligase Reaction Buffer; for its specific formulation, please visit the websites of NEB or refer to https://www.neb.com/products/restriction-endonucleases or https://www.neb.com/products/b0202-t4-dna-ligase-reaction-buffer) and 0.5 µL of T₄-DNA ligase, and then ligated overnight at 4°C. The 5' and 3' end genomic DNAs were isolated by PCR amplification using a series of nested primers. Specifically, the primer combination for isolating the 5' end genomic DNA comprises SEQ ID NO: 13 and SEQ ID NO: 30 as first primers, SEQ ID NO: 32 and SEQ ID NO: 33 as second primers, and SEQ ID NO: 13 as a sequencing primer. The primer combination for isolating 3' end genomic DNA comprises SEQ ID NO: 15 and SEQ ID NO: 31 as first primers, SEQ ID NO: 34 and SEQ ID NO: 35 as second primers, and SEQ ID NO: 15 as a sequencing primer. PCR reaction conditions were shown in Table 3.

The amplification product resulted from the above PCR amplification was subject to electrophoresis on an agarose gel with a mass fraction of 2.0% to isolate the PCR amplification product; subsequently, the target fragment was isolated from agarose matrix using a gel extraction kit (QIAquick Gel Extraction Kit, catalog #_28704, Qiagen Inc., Valencia, CA). Then the purified PCR amplification product was sequenced (e.g., using ABI PrismTM 377, PE Biosystems, Foster City, CA) and analyzed (e.g., using DNASTAR sequence analysis software, DNASTAR Inc., Madison, WI).

The 5' and 3' flanking sequences and junction sequences were confirmed using standard PCR procedures. The 5' flanking and junction sequences were confirmed using SEQ ID NO: 8 or SEQ ID NO: 12 in combination with SEQ ID NO: 9, SEQ ID NO: 13 or SEQ ID NO: 30. The 3' flanking and junction sequences were confirmed using SEQ ID NO: 11 or SEQ ID NO: 14 in combination with SEQ ID NO: 10, SEQ ID NO: 15 or SEQ ID NO: 31. PCR reaction systems and amplification conditions were shown in Tables 2 and 3. It will be recognized by those skilled in the art that other primer sequences could also be used to confirm the flanking and junction sequences.

DNA sequencing of the PCR amplification products provides a DNA that could be used to design other DNA molecules which could be used as primers and probes for identifying soybean plants or seeds derived from the transgenic soybean event DBN8205.

The inserted sequence of the transgenic soybean event DBN8205 was found to be flanked on the right border (5' flanking sequence) by the soybean genomic sequence shown in the nucleotides 1-481 of SEQ ID NO: 5 and flanked on the left border (3' flanking sequence) by the soybean genomic sequence shown in the nucleotides 12397-12813 of SEQ ID NO: 5. The 5' junction sequence was set forth in SEQ ID NO: 1 and the 3' junction sequence was set forth in SEQ ID NO: 2.

### 3.3. PCR assay for zygosity

Junction sequence is a relatively short polynucleotide molecule, which is a new DNA sequence and is diagnostic for the DNA of the transgenic soybean event DBN8205 when detected in polynucleotide detection and analysis. The junction sequences in SEQ ID NO: 1 and SEQ ID NO: 2 respectively are 11 polynucleotides on each side of the insertion site of the transgenic fragment and soybean genomic DNA in the transgenic soybean event DBN8205. Longer or shorter polynucleotide junction sequences can be selected from SEQ ID NO: 3 or SEQ ID NO: 4. The junction sequences (5' junction region, SEQ ID NO: 1 and 3' junction region, SEQ ID NO: 2) were useful in the method for detecting DNA as DNA probes or DNA primer molecules. The junction sequences SEQ ID NO: 6 and SEQ ID NO: 7 were also new DNA sequences of the transgenic soybean event DBN8205, and could also be used for detecting the presence of the transgenic soybean event DBN8205 as DNA probes or DNA primer molecules. SEQ ID NO: 6 spans a DNA sequence of the pDBN4031 construct and a prAtAct2-01 transcription origin sequence, and SEQ ID NO: 7 spans a t35S transcriptional termination sequence and a DNA sequence of the pDBN4031 construct.

Furthermore, an amplicon was generated by using at least one primer derived from SEQ ID NO: 3 or SEQ ID NO: 4. Said primer, when used in a PCR method, generated an amplicon diagnostic for the transgenic soybean event DBN8205.

Specifically, the PCR amplification product was generated from the 5' end of the transgenic inserted sequence. Said PCR amplification product comprises a portion of the genomic DNA at 5' end flanking the T-DNA inserted sequence in the genome of plant materials derived from the transgenic soybean event DBN8205. The PCR amplification product comprises SEQ ID NO: 3. For PCR amplification, primer 7 (SEQ ID NO: 8), which may hybridize to a genomic DNA sequence at 5' end flanking the transgenic inserted sequence, and primer 8 (SEQ ID NO: 9), which is in pair with primer 7 and located in the prAtAct2-01 transcription origin sequence of the T-DNA inserted sequence, were designed.

The PCR amplification product was generated from the 3' end of the transgenic inserted sequence. Said PCR amplification product comprises a portion of the genomic DNA at 3' end flanking the T-DNA inserted sequence in the genome of plant materials derived from the transgenic soybean event DBN8205. The PCR amplification product comprises SEQ ID NO: 4. For PCR amplification, primer 9 (SEQ ID NO: 10), which is located in the t35S transcriptional termination sequence of the T-DNA inserted sequence, and primer 10 (SEQ ID NO: 11), which is in pair with primer 9 and may hybridize to a genomic DNA sequence at 3' end flanking the transgenic inserted sequence, were designed.

DNA amplification conditions described in Tables 2 and 3 could be used in the above PCR assays for zygosity to generate an amplicon diagnostic for the transgenic soybean event DBN8205. Detection of amplicons could be conducted by using a thermocycler such as Stratagene Robocycler, MJ Engine, Perkin-Elmer 9700, or Eppendorf Mastercycler Gradient, or by the methods and apparatus known to those skilled in the art.

**Table 2: The steps and conditions of reaction mixture in the PCR reaction for the identification of the 5' end transgenic insert/genomic junction region of the transgenic soybean event DBN8205**

| Step | Reagent | Amount | Note |
|---|---|---|---|
| 1 | Nuclease-free water | Added to a final volume of 20 µL | |
| 2 | 10 × reaction buffer (with MgCl₂) | 2.0 µL | The final concentration of 1 × buffer; the final concentration of MgCl₂: 1.5 mM |
| 3 | 10 mM solution of dATP, dCTP, dGTP and dTTP | 0.2 µL | The final concentration of each dNTP: 200 µM |
| 4 | Primer 7 (SEQ ID NO: 8) resuspended in 1 × TE buffer or nuclease-free water to a concentration of 10 µM | 0.2 µL | The final concentration: 0.1 µM |
| 5 | Primer 8 (SEQ ID NO: 9) resuspended in 1 × TE buffer or nuclease-free water to a concentration of 10 µM | 0.2 µL | The final concentration: 0.1 µM |
| 6 | RNase; DNase-free (500 µg/ml) | 0.1 µL | 50 ng/reaction |
| 7 | *REDTaq*^{®} DNA polymerase (1 unit/µL) | 1.0 µL (recommended to shift a pipette prior to next step) | 1 unit/reaction |
| | Extracted DNA (Template): leaves of the samples to be analyzed | 200 ng of genomic DNA | |
| | Negative control | 50 ng of non-transgenic soybean genomic DNA | |
| | Negative control | No template DNA (the solution into which DNA resuspends) | |
| | Positive control | 50 ng of soybean genomic DNA comprising DBN8205 | |

**Table 3: Amplification conditions of thermocycler**

| Cycle No. | Setting |
|---|---|
| 1 | 94°C 3 min |
| 34 | 94°C 30 sec |
| | 64°C 30 sec |
| | 72°C 1 min |
| 1 | 72°C 10 min |

The reaction solutions were gently mixed, and if there is no hot top on the thermocycler, added with 1-2 drops of mineral oil on the top of each reaction solution. PCR reaction was conducted in a thermocycler such as Stratagene Robocycler (Stratagene, La Jolla, CA), MJ Engine (MJ R-Biorad, Hercules, CA), Perkin-Elmer 9700 (Perkin Elmer, Boston, MA), or Eppendorf Mastercycler Gradient (Eppendorf, Hamburg, Germany) using the cycle parameters shown in Table 3. MJ Engine or Eppendorf Mastercycler Gradient thermocycler should be run in the calculated mode. The Perkin-Elmer 9700 thermocycler should have a ramp speed set at maximum when running.

The experiments show the following results: when used in a PCR reaction of the transgenic soybean event DBN8205 genomic DNA, primers 7 and 8 (SEQ ID NO: 8 and 9) result in a 634 bp fragment of an amplification product, but when they were used in PCR reactions of a non-transformed soybean genomic DNA and non-DBN8205 soybean genomic DNA, no fragment was amplified; when used in a PCR reaction of the transgenic soybean event DBN8205 genomic DNA, primers 9 and 10 (SEQ ID NO: 10 and 11) result in a 642 bp fragment of an amplification product, but when they were used in PCR reactions of a non-transformed soybean genomic DNA and non-DBN8205 soybean genomic DNA, no fragment was amplified.

PCR assays for zygosity could also be used to identify whether the material derived from the transgenic soybean event DBN8205 is homozygous or heterozygous. Primer 11 (SEQ ID NO: 12), primer 12 (SEQ ID NO: 13) and primer 13 (SEQ ID NO: 14) were used in an amplification reaction to produce an amplicon diagnostic for the transgenic soybean event DBN8205. DNA amplification conditions described in Tables 4 and 5 could be used in the above assays for zygosity to produce an amplicon diagnostic for the transgenic soybean event DBN8205.

**Table 4: Reaction solution for the assays for zygosity**

| Step | Reagent | Amount | Note |
|---|---|---|---|
| 1 | Nuclease-free water | Added to a final volume of 5 µl | |
| 2 | 2 × Universal Master Mix (Applied Biosystems Catalog No. 4304437) | 2.5 µL | The final concentration: 1 × |
| 3 | Primer 11 (SEQ ID NO: 12) and primer 12 (SEQ ID NO: 13) resuspended in nuclease-free water to a concentration of 10 µM | 0.05 µL | The final concentration: 0.25 µM |
| 4 | Primer 13 (SEQ ID NO: 14) resuspended in 1 × TE buffer or nuclease-free water to a concentration of 10 µM | 0.01 µL | The final concentration: 0.15 µM |
| 5 | *REDTaq*^{®} DNA polymerase (1 unit/µl) | 1.0 µl (recommended to shift a pipette prior to next step) | 1 unit/reaction |
| 6 | Extracted DNA (template): leaves of the samples to be analyzed | 200 ng of genomic DNA | |
| | Negative control | 50 ng of non-transgenic soybean genomic DNA | |
| | Negative control | No template DNA (the solution into which DNA resuspends) | |
| | Positive control | 50 ng of soybean genomic DNA comprising DBN8205 | |

**Table 5: Thermocycler amplification conditions for the assays for zygosity**

| Cycle No. | Setting |
|---|---|
| 1 | 95°C 10 min |
| 10 | 95°C 15 sec |
| | 64°C 1 min (-1°C/cycle) |
| 30 | 95°C 15 sec |
| | 54°C 1 min |
| 1 | 10°C immersion |

PCR reaction was conducted in a thermocycler such as Stratagene Robocycler (Stratagene, La Jolla, CA), MJ Engine (MJ R-Biorad, Hercules, CA), Perkin-Elmer 9700 (Perkin Elmer, Boston, MA), or Eppendorf Mastercycler Gradient (Eppendorf, Hamburg, Germany) using the cycle parameters shown in Table 5. MJ Engine or Eppendorf Mastercycler Gradient thermocycler should be run in the calculated mode. The Perkin-Elmer 9700 thermocycler should have a ramp speed set at maximum when running.

In the amplification reaction, the biological sample comprising template DNA contains the DNA diagnostic for the presence of the transgenic soybean event DBN8205. Alternatively, the amplification reaction would generate two different DNA amplicons from the biological sample comprising the DNA derived from soybean genome, and the DNA derived from soybean genome is heterozygous for the corresponding allele of the inserted DNA present in the transgenic soybean event DBN8205. The two different amplicons would correspond to a first amplicon (SEQ ID NO: 12 and SEQ ID NO: 14) derived from a locus of wild-type soybean genome and a second amplicon (SEQ ID NO: 12 and SEQ ID NO: 13) diagnostic for the presence of the transgenic soybean event DBN8205 DNA. A soybean DNA sample that only generates a single amplicon corresponding to the second amplicon as described in regard to heterozygous genome could be diagnosed as the presence of the transgenic soybean event DBN8205 in that sample, and the sample was produced from soybean seeds homozygous for the corresponding allele of the inserted DNA present in the transgenic soybean plant DBN8205.

It should be noted that the primer pairs for the transgenic soybean event DBN8205 are used to produce an amplicon diagnostic for genomic DNA of the transgenic soybean event DBN8205. These primer pairs include but are not limited to primers 7 and 8 (SEQ ID NO: 8 and SEQ ID NO: 9), and primers 9 and 10 (SEQ ID NO: 10 and SEQ ID NO: 11), and are used in the DNA amplification method. In addition, control primers 14 and 15 (SEQ ID NOs: 25 and 26) for amplification of an endogenous soybean gene are included as an internal standard for reaction conditions. The analysis of a DNA extraction sample of the transgenic soybean event DBN8205 should include a positive tissue DNA extract from the transgenic soybean event DBN8205 as control, a negative DNA extract from a soybean plant that is not the transgenic soybean event DBN8205 as control, and a negative control without template soybean DNA extract. In addition to these primer pairs, any primer pair from SEQ ID NO: 3 or a complementary sequence thereof, or SEQ ID NO: 4 or a complementary sequence thereof, can be used; when they are used for DNA amplification reaction, they respectively produce amplicons comprising SEQ ID NO: 1 or SEQ ID NO: 2 and diagnostic for tissues derived from the transgenic soybean event DBN8205. DNA amplification conditions as described in Tables 2 to 5 can be used in the production of an amplicon diagnostic for the transgenic soybean event DBN8205 by using suitable primer pairs. The DNA extract of soybean plants or seeds which produces an amplicon diagnostic for the transgenic soybean event DBN8205 in a DNA amplification method and is presumed to contain the transgenic soybean event DBN8205, or a product derived from the transgenic soybean event DBN8205 can be used as a template for amplification to determine the presence of the transgenic soybean event DBN8205.

### Example 4: Detection of the transgenic soybean event DBN8205 by Southern blot

### 4.1 DNA extraction for use in Southern blot

Approximately 5 to 10 g of plant tissue was ground in liquid nitrogen using a mortar and a pestle. 4 to 5 g of the ground plant tissue was resuspended in 20 mL of CTAB lysis buffer (100 mM Tris-HCl pH 8.0, 20 mM EDTA pH 8.0, 1.4 M NaCl, 0.2% v/v β-mercaptoethanol, 2% w/v CTAB), and incubated at a temperature of 65°C for 60 minutes. During incubation, the sample was uniformly mixed by inversion once per 10 minutes. After incubation, an equal volume of phenol/chloroform/isoamyl alcohol (25:24:1) was added, and gently mixed by inversion for extraction, and then centrifuged at a rotation speed of 4,000 rpm for 20 minutes. The aqueous phase was collected and re-extracted once with an equal volume of chloroform/isoamyl alcohol (24:1). The aqueous phase was re-collected and added with an equal volume of isopropanol. The mixture was mixed uniformly, left standing at a temperature of -20°C for 1 hour to precipitate DNA, and then centrifuged at a rotation speed of 4,000 rpm for 5 minutes to obtain the DNA pellet, which was subsequently resuspended in 1 mL of TE buffer (10 mM Tris-HCl, 1 mM EDTA; pH 8.0). In order to degrade any possible RNA, the DNA was incubated with 40 µL of 10 mg/mL RNase A at a temperature of 37°C for 30 minutes, centrifuged at a rotation speed of 4,000 rpm for 5 minutes, and then centrifuged at a rotation speed of 12,000 rpm for 10 minutes in the presence of 0.1-fold volume of 3 M sodium acetate (pH 5.2) and 2-fold volumes of absolute ethanol to precipitate DNA. After the supernatant was discarded, the pellet was washed with 1 mL of 70% (v/v) ethanol and dried under room temperature, and then the DNA was re-dissolved in 1 mL of TE buffer.

### 4.2 Restriction enzyme digestion

The concentration of genomic DNA in the above-mentioned sample was measured by using an ultra micro-volume spectrophotometer (NanoDrop 2000, Thermo Scientific).

In a 100 µL of reaction system, 5 µg DNA was digested each time. Genomic DNA was digested respectively with restriction endonucleases *Mfe* I, *Nco* I, *Hind* III and *Sph* I using a portion of the sequences of cCry2Ab gene, cCry1Ac gene and cPAT gene in T-DNA as probes. For each enzyme, the digestion products were incubated overnight at an appropriate temperature. The sample was spun in a centrifugal vacuum concentrator (speed Vacuum, Thermo Scientific) to reduce the volume to 20 µl.

### 4.3 Gel electrophoresis

Bromophenol blue loading dye was added to each sample from Example 4.2, and each sample was loaded onto a 0.7% agarose gel containing ethidium bromide and electrophoresed for isolation in TAE electrophoresis buffer (40 mM Tris-acetic acid, 2 mM EDTA, pH 8.5). The gel electrophoresis was run overnight at a voltage of 20 V

After the electrophoresis, the gel was treated with 0.25 M HCl for 10 minutes to depurinate the DNA, and then treated with a denaturation solution (1.5 M NaCl, 0.5 M NaOH) and a neutralization solution (1.5 M NaCl, 0.5 M Tris-HCl; pH 7.2) for 30 minutes, respectively. 5 × SSC (3 M NaCl, 0.3 M Sodium citrate; pH 7.0) was poured into a porcelain dish; a piece of glass was covered; and then a wet filter paper bridge, a gel, a positively charged nylon membrane (Roche, Cat. No. 11417240001), three pieces of filter paper, a paper tower, and a heavy object were placed successively. After membrane-transfer at room temperature overnight, the nylon membrane was rinsed with deionized water twice, and the DNA was immobilized onto the membrane by ultraviolet crosslinker (UVP, UV Crosslinker CL-1000).

### 4.4 Hybridization

A suitable DNA sequence was amplified by PCR for probe preparation. The DNA probes were SEQ ID NO: 27, SEQ ID NO: 28 or SEQ ID NO: 29, or partially homologous or complementary to the above sequences. The DIG labeling of the probes, Southern blot, membrane washing, and other operations were performed by using the DNA Labeling and Detection Starter Kit II (Roche, Cat. No. 11585614910). For specific methods, please refer to their Product Instructions. Finally, an X-ray film (Roche, Cat. No. 11666916001) was used to detect the position where the probe was bound.

Each Southern blot included two control samples: (1) DNA from a negative (untransformed) segregant, which was used to identify any endogenous soybean sequences that could hybridize to the element-specific probe; and (2) DNA from a negative segregant, into which *Hind* III-digested pDBN4031 plasmid was introduced, in an amount equivalent to a single copy number based on the length of the probe, which was used as a positive control to show the sensitivity of the experiment when a single copy of gene was detected in the soybean genome.

The hybridization data provides corroboratory evidence to support TaqMan^{™} PCR analysis, namely, the soybean plant DBN8205 contains cCry2Ab gene, cCry1Ac gene and cPAT gene in a single copy. By using the probe for cCry2Ab gene, the enzymatic digestions of *Mfe* I and *Spe* I respectively generate single bands of about 8.0 kb and 6.0 kb; and by using the probe for cCry1Ac gene, the enzymatic digestions of *Hind III* and *Sph I* respectively generate single bands of about 13 kb and 7.5 kb; and by using the probe for cPAT gene, the enzymatic digestions of *Mfe I* and *Spe I* respectively generate single bands of about 9.5 kb and 13 kb. This indicates that the cCry2Ab gene, cCry1Ac gene and cPAT gene were respectively present in the soybean plant DBN8205 in one copy. Furthermore, no hybridization band was obtained using the backbone probe, indicating that no backbone sequence of the vector pDBN4031 was introduced into the genome of the soybean plant DBN8205 during transformation.

### Example 5: Detection of protein expression in the transgenic soybean event DBN8205 by ELISA

The expression ranges of Cry1Ac protein in the transgenic soybean event DBN8205 can be detected by ELISA.

The leaves at V5 stage, stems and flowers at R2 stage, roots, stems and seeds at R6 stage were extracted from transgenic soybean event DBN8205, and the different soybean tissues at different growth stages described above were lyophilized and used as the samples. 20 mg of samples were weighed respectively and ground in liquid nitrogen, then added with 1 mL extraction buffer (8 g/L NaCl, 0.27 g/L KH₂PO₄, 1.42 g/L Na₂HPO₄, 0.2 g/L KCl, and 5.5 ml/L Tween-20; pH 7.4) respectively. The mixture was mixed uniformly, left standing at a temperature of 4°C for 30 minutes, and then centrifuged at a rotation speed of 12,000 rpm for 10 minutes. The supernatant was pipetted and diluted with the above extraction buffer to suitable dilution. 80 µL of the diluted supernatant was collected for detection by ELISA.

The proportion of protein (Cry1Ac protein) amounts in the sample based on the dry weight of different soybean tissues was measured and analyzed by ELISA (Enzyme-Linked Immunosorbent Assay) detection kits (ENVIRLOGIX Company, Cry1Ac kit (AP003)). For specific methods, please refer to their Product Instructions. Meanwhile, corresponding tissues of a wild-type soybean plant (non-transgenic, NGM) were used as a control sample. Detection and analysis were carried out according to the above-mentioned method, with 6 replicates per plant.

The experimental results of the Cry1Ac protein contents of the transgenic soybean event DBN8205 were as shown in Table 6. The proportion of average expression level of Cry1Ac protein in the transgenic soybean event DBN8205 in different tissues at different growth stages based on the dry weight of the corresponding tissues was determined to be 1.11 to 342.05 (µg/g).

**Table 6 Results of the Cry1Ac protein expression levels (µg/g) in the transgenic soybean event DBN8205**

| Growth stage | Tissue | Cry1Ac protein expression level (µg/g) | NGM |
|---|---|---|---|
| V5 stage | Leaf | 342.05±26.09 | not detected |
| R2 stage | Stem | 5.3±1.97 | not detected |
| | Flower | 12.45±0.76 | not detected |
| R6 stage | Root | 1.11±0.08 | not detected |
| | Stem | 5.24±1.87 | not detected |
| | Seed | 8.44±3.23 | not detected |

The results of Table 6 show that in the transgenic soybean event DBN8205, the proportion of average expression level of Cry1Ac protein based on dry weight of the soybean leaves at V5 stage was 342.05µg/g, the proportion of average expression level of Cry1Ac protein based on dry weight of the soybean stems at R2 stage was 5.3µg/g, the proportion of average expression level of Cry1Ac protein based on dry weight of the soybean flowers at R2 stage was 12.45µg/g, the proportion of average expression level of Cry1Ac protein based on dry weight of the soybean roots at R6 stage was 1.11µg/g, the proportion of average expression level of Cry 1 Ac protein based on dry weight of the soybean seeds at R6 stage was 8.44µg/g. Table 6 sufficiently indicates that in the transgenic soybean event DBN8205, Cry1Ac proteins are expressed in different soybean tissues at different growth stages, and have higher expression levels especially in leaves, flowers and seeds, which shows good resistance against *Lepidoptera* insects, meanwhile indicates that the vector pDBN4031 is designed excellently.

### Example 6: Evaluation of resistance of vectors pDBN4031 and pDBN4032 against insects

Seven kinds of plants, including three excellent transgenic soybean plants DBN8205, pDBN4031-1 and pDBN4031-2 screened by vector pDBN4031 and three transgenic soybean plants pDBN4032-1, pDBN4032-2 and pDBN4032-3 screened by vector pDBN4032 and wild-type soybean plant (non-transgenic, NGM), were tested against the major pests (*Spodoptera exigua* (BAW) and *Spodoptera litura* (TCW)) in China according to the following bioassays:
Top second leaves at V3 stage from seven kinds of plants, i.e. the transgenic soybean event described above and wild-type soybean plant (non-transgenic, NGM), were taken respectively, rinsed with sterile water and aspirated to dryness with a gauze. Then with the removal of veins, soybean leaves were cut into shapes of about 2.5 cm × 3 cm. 1 to 3 cut leaves (the number of the leaf was determined based on the ingestion amount of insects) were placed on filter paper moistened with distilled water at the bottom of round plastic Petri dishes. 10 newly-hatched larvae by artificial feeding were placed in each dish. Then the Petri dishes were covered with lids and kept for 3 days under the following conditions: a temperature of 26-28°C, a relative humidity of 70%-80%, and a photoperiod (light/dark) of 16:8. Then a statistic result was obtained. Three indicators, larvae development rate, mortality of tested insects and leaf damage ratio, were statistically analyzed to obtain a total resistance score (full score: 300 points). The total resistance score = 100 × mortality + [100 × mortality + 90 × (number of newly-hatched larvae/a total number of the inoculated larvae) + 60 × (number of insects bigger than the size of newly-hatched larvae to smaller than the size of negative control/a total number of the inoculated larvae) + 10 × (number of insects for negative control/a total number of the inoculated larvae)] + 100 × (1 - leaf damage ratio), wherein a total number of the inoculated larvae refers to the total number of the larvae inoculated, i.e. 10 larvae in each dish; the larvae development rate was reflected by the formula of the total resistance score; and the leaf damage ratio refers to the proportion of pest-ingested area in total leaf area. For each insect, five plants from the transgenic soybean plants DBN8205, pDBN4031-1, pDBN4031-2, pDBN4032-1, pDBN4032-2, pDBN4032-3 and the wild-type soybean plant (non-transgenic, NGM) were tested respectively, 3 replicates per plant. The results were shown in Tables 7.

**Table 7: The bioassay results of insect resistance of the transgenic soybean plants DBN8205, pDBN4031-1, pDBN4031-2, pDBN4032-1, pDBN4032-2, pDBN4032-3 - Mortality (%) and total resistance score (points)**

| Vector | Transformation event | BAW | | TCW | |
|---|---|---|---|---|---|
| | | Mortality (%) | Total resistance score | Mortality (%) | Total resistance score |
| pDBN4031 | DBN8205 | 100±0 | 299±0 | 100±0 | 299±0 |
| | pDBN4031-1 | 97±2 | 294±4 | 98±2 | 296±3 |
| | pDBN4031-2 | 97±2 | 294±4 | 100±0 | 299±0 |
| pDBN4032 | pDBN4032-1 | 87±5 | 278±8 | 81±5 | 282±7 |
| | pDBN4032-2 | 84±6 | 258±11 | 76±9 | 253±10 |
| | pDBN4032-3 | 84±7 | 258±10 | 81±6 | 282±7 |
| NGM | | 6±5 | 62±10 | 10±0 | 86±14 |

The results of Table 7 show that (1) transgenic soybean events (DBN8205, pDBN4031-1 and pDBN4031-2) screened by vector pDBN4031 and transgenic soybean events (pDBN4032-1, pDBN4032-2 and pDBN4032-3) screened by vector pDBN4032 exhibit significantly more excellent resistance against *Spodoptera exigua* and *Spodoptera litura* than the NGM; (2) transgenic soybean events (DBN8205, pDBN4031-1 and pDBN4031-2) screened by vector pDBN4031 exhibit more excellent resistance against *Spodoptera exigua* and *Spodoptera litura* than transgenic soybean events (pDBN4032-1, pDBN4032-2 and pDBN4032-3) screened by vector pDBN4032, indicating that the vector pDBN4031 is designed more excellently, which is an excellent vector obtained by sufficiently considering and analyzing combinations and interactions of regulatory elements; (3) the resistance of transgenic soybean event DBN8205 against *Spodoptera exigua* and *Spodoptera litura* is the most excellent.

### Example 7: Detection of the resistance of the event DBN8205 against insects

For further verifing effect of resistance of DBN8205 event against insects, major pests in China and South America (Argentina and Brazil) were selected to carry out bioassay experiments and field efficacy experiments.

### 7.1 Bioassay of the soybean plant DBN8205 against major pests in China

Two kinds of plants, i.e. the transgenic soybean event DBN8205 and wild-type soybean plant (non-transgenic, NGM), were tested against *Helicoverpa armigera* (CBW), *Agrotis ypsilon* (BCW), *Clanis bilineata* (BHM) and *Spodoptera frugiperda* (FAW) according to the following bioassays:
Top second leaves at V3 stage from two kinds of plants, i.e. the transgenic soybean event DBN8205 and wild-type soybean plant (non-transgenic, NGM), were taken respectively, rinsed with sterile water and aspirated to dryness with a gauze. Then with the removal of veins, soybean leaves were cut into shapes of about 2.5 cm × 3 cm. 1 to 3 cut leaves (the number of the leaf was determined based on the ingestion amount of insects) were placed on filter paper moistened with distilled water at the bottom of round plastic Petri dishes. 10 newly-hatched larvae by artificial feeding were placed in each dish. Then the Petri dishes were covered with lids and kept for 3 days under the following conditions: a temperature of 26-28°C, a relative humidity of 70%-80%, and a photoperiod (light/dark) of 16:8. Then a statistic result was obtained. Three indicators, larvae development rate, mortality of tested insects and leaf damage ratio, were statistically analyzed to obtain a total resistance score (full score: 300 points). The total resistance score = 100 × mortality + [100 × mortality + 90 × (number of newly-hatched larvae/a total number of the inoculated larvae) + 60 × (number of insects bigger than the size of newly-hatched larvae to smaller than the size of negative control/a total number of the inoculated larvae) + 10 × (number of insects for negative control/a total number of the inoculated larvae)] + 100 × (1 - leaf damage ratio), wherein a total number of the inoculated larvae refers to the total number of the larvae inoculated, i.e. 10 larvae in each dish; the larvae development rate was reflected by the formula of the total resistance score; and the leaf damage ratio refers to the proportion of pest-ingested area in total leaf area. For each insect, five plants from the transgenic soybean event DBN8205 and the wild-type soybean plant (non-transgenic, NGM) were tested respectively, 6 replicates per plant. The results were shown in Tables 8.

**Table 8: The bioassay result of insect resistance of the transgenic soybean event DBN8205 against major pests in China - Mortality (%) and total resistance score (points)**

| Insect/plant | DBN8205 | | NGM | |
|---|---|---|---|---|
| | Mortality (%) | Total resistance score | Mortality (%) | Total resistance score |
| CBW | 100±0 | 299±0 | 15±6 | 84±16 |
| BCW | 56±15 | 227±33 | 18±8 | 99±18 |
| BHM | 93±7 | 289±10 | 8±8 | 33±23 |
| FAW | 34±5 | 178±23 | 0±0 | 24±5 |

The results show that the transgenic soybean event DBN8205 exhibits significantly higher mortality of tested insects and total resistance score than the NGM, indicating that the transgenic soybean event DBN8205 has good resistance against *Helicoverpa armigera, Agrotis ypsilon, Clanis bilineata* and *Spodoptera frugiperda.*

### 7.2 Field test of the transgenic soybean event DBN8205 in China

The transgenic soybean event DBN8205 and wild-type soybean plant (non-transgenic, NGM) were planted in a field. Randomized block design with 3 replicates was employed. The district had an area of 30 m² (5 m × 6 m), row spacing of 60 cm and plant spacing of 10 cm. The plants were conventionally cultivated and managed, and were not sprayed with insecticides against the target pests during the whole propagation period.

### (1) Helicoverpa armigera

Natural infestation was performed only in the area with serious natural occurrence of *Helicoverpa armigera* (the conditions for the occurrence of natural insect damage: the most severe damage occurs from June to July and the optimum temperature for development ranges from 20 to 30°C). When the soybean plants grew to V2 stage, the conditions of NGM leaves ingested by larvae of *Helicoverpa armigera* were tracked and investigated. When the top second and third leaves of the NGM were not ingested, the damage area rate (the damage area rate = the sum of the leaf damage area in all single plants/the total area of plant leaves × 100%) of soybean plants by *Helicoverpa armigera* was investigated one by one. The result of resistance of the transgenic soybean event DBN8205 against *Helicoverpa armigera* is shown in Table 9.

**Table 9: The result of resistance of the transgenic soybean event DBN8205 against Helicoverpa armigera under the conditions of natural infestation**

| Program/plant | DBN8205 | NGM |
|---|---|---|
| Damage area rate (%) | 0.0±0.2 | 11.4±5.5 |

The results show that under the conditions of natural occurrence of *Helicoverpa armigera,* as compared with the NGM, the damage area rate of transgenic soybean event DBN8205 by *Helicoverpa armigera* is 0, indicating that *Helicoverpa armigera* causes basically no damage to the leaves of transgenic soybean event DBN8205, and that the transgenic soybean event DBN8205 has good resistance against *Helicoverpa armigera.* The field effect of the transgenic soybean event DBN8205 under the conditions of natural occurrence of *Helicoverpa armigera* is shown in Figure 4.

### (2) Spodoptera exigua

Natural infestation was performed only in the area with serious natural occurrence of *Spodoptera exigua* (the conditions for the occurrence of natural insect damage: the most severe damage occurs from June to July and the optimum temperature for development ranges from 20 to 30°C). When the soybean plants grew to V2 stage, the conditions of NGM leaves ingested by larvae of *Spodoptera exigua* were tracked and investigated. When the top second and third leaves of the NGM were not ingested, the damage area rate (the damage area rate = the sum of the leaf damage area in all single plants/the total area of plant leaves × 100%) of soybean plants by *Spodoptera exigua* was investigated one by one. The result of resistance of the transgenic soybean event DBN8205 against *Spodoptera exigua* is shown in Table 10.

**Table 10: The result of resistance of the transgenic soybean event DBN8205 against Spodoptera exigua under the conditions of natural infestation**

| Program/plant | DBN8205 | NGM |
|---|---|---|
| Damage area rate (%) | 2.1±0.3 | 8.6±0.6 |

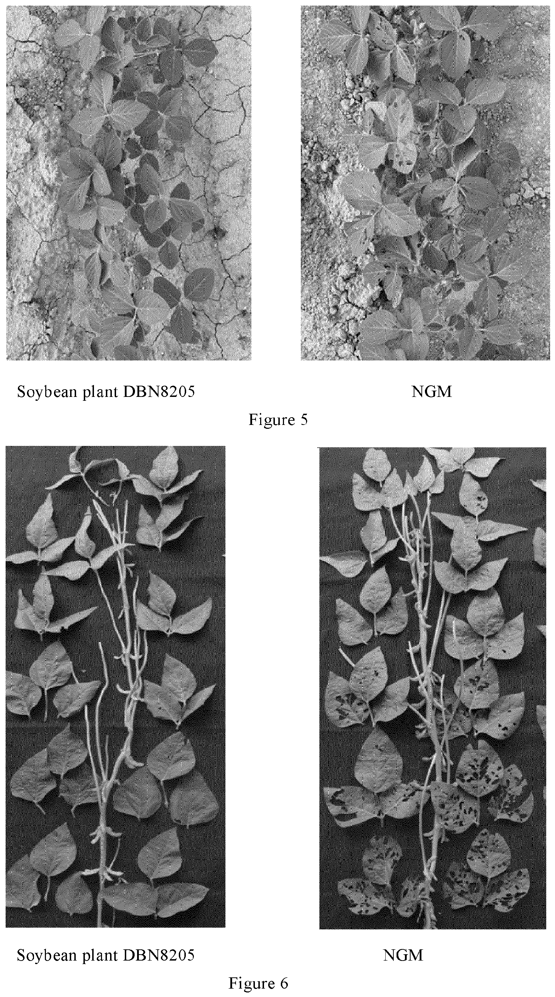

The results show that under the conditions of natural occurrence of *Spodoptera exigua,* as compared with the NGM, the transgenic soybean event DBN8205 exhibits significantly reduced damage area rate by *Spodoptera exigua,* thus indicating that the transgenic soybean event DBN8205 has good resistance against *Spodoptera exigua.* The field effect of the transgenic soybean event DBN8205 under the conditions of natural occurrence of *Spodoptera exigua* is shown in Figure 5.

### (3) Argyrogramma agnata

Natural infestation was performed only in the area with serious natural occurrence of *Argyrogramma agnata* (the conditions for the occurrence of natural insect damage: the most severe damage occurs from June to September and the optimum temperature for development ranges from 20 to 30°C). When the soybean plants grew to V2 stage, the conditions of NGM leaves ingested by larvae of *Argyrogramma agnata* were tracked and investigated. When the top second and third leaves of the NGM were not ingested, the damage area rate (the damage area rate = the sum of the leaf damage area in all single plants/the total area of plant leaves × 100%) of soybean plants by *Argyrogramma agnata* was investigated one by one. The result of resistance of the transgenic soybean event DBN8205 against *Argyrogramma agnata* is shown in Table 11.

**Table 11: The result of resistance of the transgenic soybean event DBN8205 against Argyrogramma agnata under the conditions of natural infestation**

| Program/plant | DBN8205 | NGM |
|---|---|---|
| Damage area rate (%) | 0.2±0.6 | 16.2±6.4 |

The results show that under the conditions of natural occurrence of *Argyrogramma agnata,* as compared with the NGM, the transgenic soybean event DBN8205 exhibits significantly reduced damage area rate by *Argyrogramma agnata,* thus indicating that the transgenic soybean event DBN8205 has good resistance against *Argyrogramma agnata.* The comparison of effects between the transgenic soybean event DBN8205 and NGM under the condition of natural occurrence of *Argyrogramma agnata* is shown in Figure 6.

### (4) Leguminivora glycinivorella

Natural infestation was performed only in three areas with serious natural occurrence of *Leguminivora glycinivorella* in China (the conditions for the occurrence of natural insect damage: the most severe damage occurs from August to September and the optimum temperature for development ranges from 20 to 25°C). When the soybean plants grew to R6 stage, the conditions of NGM pods ingested by larvae of *Leguminivora glycinivorella* were tracked and investigated; when the plants were fully mature, rate of ingested grain (rate of ingested grain = sum of the number of grains damaged by insects in all individual pods/the total number of grains in plant pods × 100%) of soybean pods was investigated one by one. The result of resistance of the transgenic soybean event DBN8205 against *Leguminivora glycinivorella* is shown in Table 12.

**Table 12: The result of resistance of the transgenic soybean event DBN8205 against Leguminivora glycinivorella under the conditions of natural infestation**

| Area | Program/plant | rate of ingested grain (%) |
|---|---|---|
| Area one | DBN8205 | 0.0±0.0 |
| | NGM | 16.2±2.0 |
| Area two | DBN8205 | 0.0±0.0 |
| | NGM | 7.9±2.0 |
| Area three | DBN8205 | 0.0±0.0 |
| | NGM | 4.1±0.8 |

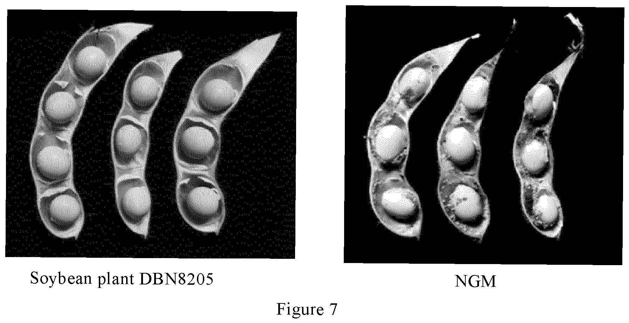

The results show that under the conditions of natural occurrence of *Leguminivora glycinivorella,* as compared with the NGM, the rate of ingested grain of transgenic soybean event DBN8205 by *Leguminivora glycinivorella* is 0, indicating that *Leguminivora glycinivorella* causes basically no damage to the pods of transgenic soybean DBN8205, and that the transgenic soybean event DBN8205 has good resistance against *Leguminivora glycinivorella.* The field effect in area one of the transgenic soybean event DBN8205 under the conditions of natural occurrence of *Leguminivora glycinivorella* is shown in Figure 7.

### 7.3 Bioassay of the soybean plant DBN8205 against major pests in South America (Argentina and Brazil)

### (1) Bioassay of the soybean plant DBN8205 against major leaf-eating pests in South America (Argentina and Brazil)

Two kinds of plants, i.e. the transgenic soybean event DBN8205 and wild-type soybean plant (non-transgenic, NGM), were tested against *Rachiplusia nu* (SFL), *Anticarsia gemmatalis* (VBC), *Chrisiodexys includes* (SBL), *Helicoverpa gelotopoeon* (SABW), *Chloridea virescens* (TBW), *Spodoptera frugiperda* (FAW), *Spodoptera cosmioides* (BLAW), *Helicoverpa zea* (SPW), *Spodoptera eridania* (SAW) and *Spodoptera albula* (GSAW) according to the following bioassays:
Top second leaves at V3 stage from two kinds of plants, i.e. the transgenic soybean event DBN8205 and wild-type soybean plant (non-transgenic, NGM), were taken respectively, rinsed with sterile water and aspirated to dryness with a gauze. Then with the removal of veins, the soybean leaves were cut into circular shapes with a diameter of about 1.6 cm. Then, 1 to 3 (the number of the leaf was determined based on the ingestion amount of insects) circular leaves were placed on round plastic petri dishes with 2mL agar. One newly-hatched larva was placed in each petri dish. Then the plates were covered with lids and kept for 3 days under the following conditions: a temperature of 26-28°C, relative humidity of 60%-80%, and a photoperiod (light/dark) of 14:10. Then a statistic result was obtained. Mortality of tested insects and leaf damage ratio (the leaf damage ratio refers to the proportion of leaf area ingested by pests in the total area of leaf) were statistically analyzed. For each pest, 6 plants with equivalent growth vigor from the transgenic soybean event DBN8205 and wild-type soybean plant (non-transgenic, NGM) were selected and tested respectively, with 32 petri dish replicates per plant. The results are shown in Table 13.

**Table 13: Bioassay of the transgenic soybean event DBN8205 against major pests in South America (Argentina and Brazil)**

| Insect | Plant | Mortality (%) | Leaf damage ratio (%) |
|---|---|---|---|
| SFL | DBN8205 | 100±0 | 3±1 |
| | NGM | 15±7 | 40±7 |
| VBC | DBN8205 | 100±0 | 3±1 |
| | NGM | 7±3 | 39±2 |
| SBL | DBN8205 | 100±0 | 3±1 |
| | NGM | 16±7 | 42±4 |
| SABW | DBN8205 | 100±0 | 3±1 |
| | NGM | 42±13 | 21±4 |
| TBW | DBN8205 | 100±0 | 3±1 |
| | NGM | 3±2 | 48±4 |
| FAW | DBN8205 | 56±7 | 5±1 |
| | NGM | 24±6 | 45±2 |
| BLAW | DBN8205 | 22±9 | 33±5 |
| | NGM | 8±3 | 38±5 |
| SPW | DBN8205 | 24±4 | 15±3 |
| | NGM | 15±4 | 25±6 |
| SAW | DBN8205 | 32±16 | 9±3 |
| | NGM | 1±1 | 49±4 |
| GSAW | DBN8205 | 37±9 | 8±4 |
| | NGM | 18±10 | 32±9 |

The results show that the transgenic soybean event DBN8205 exhibits significantly higher mortality of the above tested insects than the NGM, and exhibits lower leaf damage ratio than the NGM, indicating that the transgenic soybean event DBN8205 has good resistance against *Rachiplusia nu, Anticarsia gemmatalis, Chrisiodexys includes, Helicoverpa gelotopoeon, Chloridea virescens, Spodoptera frugiperda, Spodoptera cosmioides, Helicoverpa zea, Spodoptera eridania* and *Spodoptera albula.*

### (2) Effect of resistance of the soybean plant DBN8205 against soybean stem-eating pests in South America (Argentina and Brazil)

Two kinds of plants, i.e. the transgenic soybean event DBN8205 and wild-type soybean plant (non-transgenic, NGM), were tested against *Elasmopalpus lignosellus* (LSCB) according to the following bioassays to determine mortality of pests, plant damage ratio and mortality of plants.

Method for determining the mortality of tested insects: seedlings of the transgenic soybean event DBN8205 and wild-type soybean plant (non-transgenic, NGM) (32 plants respectively) germinated for three days under greenhouse conditions, were taken out with roots respectively, then placed in separate chamber plastic boxes. The bottom of the chamber plastic boxes contained 2% agar to keep the plants developing properly. Then one larva of *Elasmopalpus lignosellus* after 12h-incubation was placed on each plant. Then the chamber plastic boxes were sealed with plastic lids and kept for 5 days under the following conditions: a temperature of 23-27°C, relative humidity of 60%-80%, and a photoperiod (light/dark) of 14:10. Mortality of tested insects was statistically analyzed. Each plant includes six replicates.

Method for determining plant damage ratio and mortality of plants: the transgenic soybean event DBN8205 plant and wild-type soybean plant (non-transgenic, NGM) (32 plants respectively) emerging for seven days under greenhouse conditions were prepared. PVC pipes were placed around the pots in which the soybean plants described above were planted to add physical barriers to avoid migration of insects. Then two larvae of *Elasmopalpus lignosellus* after 12h-incubation were placed on the base of stem of each plant. Plant damage ratio (plant damage ratio refers to the proportion of the total number of survived plants damaged by pests in the total number of all tested plants) and mortality of plants (mortality of plants refers to the proportion of the total number of plants killed by pests in the total number of all tested plants) were statistically analyzed 15 days after inoculation. Each plant includes six replicates. The results are shown in Table 14.

**Table 14: Effect of resistance of the soybean plant DBN8205 against Elasmopalpus lignosellus**

| Program/plant | DBN8205 | NGM |
|---|---|---|
| mortality of insects % | 100±0 | 16±12 |
| plant damage ratio % | 3±3 | 87±10 |
| mortality of plants % | 0±0 | 7±5 |

The results show that the transgenic soybean event DBN8205 exhibits significantly higher mortality of tested insects against *Elasmopalpus lignosellus* than the NGM, and exhibits significantly lower plant damage ratio and mortality of plants than the NGM, indicating that the transgenic soybean event DBN8205 has good resistance against *Elasmopalpus lignosellus.*

### Example 8: Detection of herbicide tolerance of the event

Basta herbicide (in which the active ingredient is 18% glufosinate ammonium aqueous solution) was selected for spray application in the experiment. Randomized block design with three replicates was employed. The district had an area of 15 m² (5 m × 3 m), row spacing of 60 cm and plant spacing of 10 cm, and one-meter isolation belt was set among districts for conventional cultivation and management. The transgenic soybean event DBN8205 was treated according to the following two manners: (1) no herbicide spray application, artificial grass control to removed effect of weeds on soybean growth; and (2) spray application of Basta herbicide at V2-V3 leaf stage at a dose of 800 g a.i./ha (a.i./ha means "active ingredient per hectare"). Wild-type soybean plant (non-transgenic, NGM) was used as parallel control. It should be noted that glufosinate ammonium herbicide (such as Basta) is a contact-type herbicide; if the herbicide is manipulated inappropriately in the field, e.g., accumulating too much herbicide solution locally, phytotoxicity symptoms may occur; this does not mean that the transgenic soybean event DBN8205 has impaired tolerance; and various concentrations and dosage forms of glufosinate ammonium herbicides are also applicable to the following conclusion if they are converted to the above equivalent amount of the active ingredient glufosinate ammonium.

The investigation of phytotoxicity symptoms was respectively carried out at 1 week and 2 weeks after application, and the yields of the districts were determined at the time of harvest. The levels of phytotoxicity symptoms are shown in Table 15. The herbicide tolerance of the transformation event was assessed by using the herbicide damage rate as an indicator. In particular, the herbicide damage rate (%) = Σ (number of plants of the same damage level × level number) / (total number of plants × highest level), wherein the herbicide damage rate refers to the damage rate of glufosinate ammonium, which was determined according to the result of phytotoxicity investigation two weeks after glufosinate ammonium treatment; and the tolerance level of soybean to the herbicide was assessed based on the herbicide (glufosinate) damage rate. The soybean yield of each district was obtained by weighing the total yield (by weight) of soybean grains of 3 rows in the middle of each district. The yield difference among different treatments was measured as percentage of yield. The percentage of yield (%) = yield with spray application / yield without spray application. Results of herbicide tolerance of the transgenic soybean event DBN8205 and the soybean yield are shown in Table 16.

**Table 15: Grading standard of phytotoxicity degree of glufosinate ammonium herbicide to soybean**

| Phytotoxicity level | Description of symptoms |
|---|---|
| 1 | Regularly growing without any damage symptom |
| 2 | Slight phytotoxicity of less than 10% |
| 3 | Moderate phytotoxicity, which can be recovered later without affecting yield |
| 4 | Serious phytotoxicity, which is difficult to recover and results in reduced yield |
| 5 | Serious phytotoxicity, which cannot be recovered and results in significantly reduced yield or no yield |

**Table 16: Results of glufosinate ammonium herbicide tolerance of the transgenic soybean event DBN8205 and the soybean yield**

| Program/plant | DBN8205 | NGM |
|---|---|---|
| glufosinate ammonium damage rate% (no spray application) | 0±0 | 0 |
| glufosinate ammonium damage rate% (800 g a.i./ha) | 0±0 | 100 |
| percentage of yield % (800 g a.i./ha) | 100.8±8.3 | 0 |

The results show that, in terms of glufosinate ammonium herbicide damage rate: the damage rate of the transgenic soybean event DBN8205 treated with glufosinate ammonium herbicide (800 g a.i./ha) is 0. Thus, the transgenic soybean event DBN8205 has good tolerance to glufosinate ammonium herbicide.

In terms of the yield, there is no significant difference in the yields between the treatment with spray application of 800 g a.i./ha of glufosinate ammonium and the treatment without spray application. Thus, this further shows that the transgenic soybean event DBN8205 has good tolerance to glufosinate ammonium herbicide and the yield is not compromised.

### Example 9: Effect of resistance of the soybean plant DBN8205 in different contexts

The soybean plant DBN8205 in transformational context of soybean plant Jack in Example 1 was transferred into soybean plants in parental contexts of Heihe 43 and Zhonghuang 35 by backcross respectively, and the transgenic soybean event DBN8205 in transformational context of Heihe 43 and the transgenic soybean event DBN8205 in transformational context of Zhonghuang 35 were obtained respectively by five-generation backcross and three-generation selfing, wherein integrity of the transgenic soybean event DBN8205 was detected by PCR in each generation (see Example 3).

Six kinds of plants, i.e. soybean transformation event DBN8205 in transformational contexts of Jack, Heihe 43 and Zhonghuang 35 respectively, and wild-type soybean plant Jack, wild-type soybean plant Heihe 43 and wild-type soybean plant Zhonghuang 35 (non-transgenic, NGM), were tested against *Helicoverpa armigera* (CBW) according to the bioassays as described in 7.1 of Example 7. Each plant includes 6 replicates. The results were shown in Tables 17.

**Table 17: The bioassay result of insect resistance of the transgenic soybean plant DBN8205 in different transformational contexts - Mortality (%) and total resistance score (points)**

| Insect/plant | transformational context | DBN8205 | | NGM | |
|---|---|---|---|---|---|
| | | Mortality (%) | Total resistance score | Mortality (%) | Total resistance score |
| CBW | Jack | 100±0 | 299±0 | 15±10 | 83±32 |
| | Heihe 43 | 100±0 | 299±0 | 10±10 | 69±27 |
| | Zhonghuang 35 | 100±0 | 299±0 | 17±6 | 70±13 |

The results of Table 17 show that the transgenic soybean event DBN8205 has good resistance against *Helicoverpa armigera* in different transformational contexts, indicating that the transgenic soybean event DBN8205 in different transformational contexts has stable resistance effect against target pests.

### Example 10: Detection of the resistance of the superimposed transgenic soybean event DBN8205 × DBN8002 × DBN9004 against insects

### 1. Obtaining the superimposed transgenic soybean event DBN8205 × DBN8002 × DBN9004

The transgenic soybean event DBN9004 (CN106086011A) (male parent) was crossed with the transgenic soybean event DBN8002 (female parent) to obtain hybrid plants of the superimposed transgenic soybean event DBN8002 × DBN9004. Then two generations of selfing were performed, and the copy number of the target genes was detected by TaqMan (see Example 2) and PCR zygosity assay was performed to detect site homozygosity/heterozygosity (see Example 3), thereby obtaining the homozygous plants of the superimposed transgenic soybean event DBN8002 × DBN9004. The superimposed transgenic soybean event DBN8002 × DBN9004 as a male parent was crossed with the transgenic soybean event DBN8205 (female parent) to obtain the superimposed transgenic soybean event DBN8205 × DBN8002 × DBN9004.

### 2. Bioassay of the superimposed transgenic soybean event DBN8205 × DBN8002 × DBN9004 against major pests in China

Two kinds of plants, i.e. the superimposed transgenic soybean event DBN8205 × DBN8002 × DBN9004 and wild-type soybean plant (non-transgenic, NGM), were tested against *Helicoverpa armigera* (CBW), *Spodoptera exigua* (BAW) and *Spodoptera frugiperda* (FAW) according to the bioassays as described in 7.1 of Example 7. The results were shown in Table 18.

**Table 18: The bioassay result of the superimposed transgenic soybean event DBN8205 × DBN8002 × DBN9004 against major pests in China - Mortality (%) and total resistance score (points)**

| Insect/plant | DBN8205 × DBN8002 × DBN9004 | | NGM | |
|---|---|---|---|---|
| | Mortality (%) | Total resistance score | Mortality (%) | Total resistance score |
| CBW | 100±0 | 299±0 | 16±15 | 76±30 |
| BAW | 100±0 | 299±0 | 16±11 | 71±20 |
| FAW | 100±0 | 299±0 | 0±0 | 24±5 |

The results show that the superimposed transgenic soybean event DBN8205 × DBN8002 × DBN9004 exhibits significantly higher mortality of tested insects and total resistance score against the above pests than the NGM, indicating that the superimposed transgenic soybean event DBN8205 × DBN8002 × DBN9004 has good resistance against *Helicoverpa armigera, Spodoptera exigua* and *Spodoptera frugiperda.*

### 3. Bioassay of the superimposed transgenic soybean event DBN8205 × DBN8002 × DBN9004 against major pests in South America (Argentina and Brazil)

Two kinds of plants, i.e. the superimposed transgenic soybean event DBN8205 × DBN8002 × DBN9004 and wild-type soybean plant (non-transgenic, NGM), were tested against *Rachiplusia nu* (SFL), *Anticarsia gemmatalis* (VBC), *Chrisiodexys includes* (SBL), *Helicoverpa gelotopoeon* (SABW), *Spodoptera frugiperda* (FAW), *Spodoptera cosmioides* (BLAW), *Helicoverpa zea* (SPW) according to the following bioassays as described in 7.3 (1) of Example 7. The results were shown in Tables 19.

**Table 19: Bioassay of the superimposed transgenic soybean event DBN8205 × DBN8002 × DBN9004 against major pests in South America (Argentina and Brazil)**

| Insect | Plant | Mortality (%) | Leaf damage ratio (%) |
|---|---|---|---|
| SFL | DBN8205 × DBN8002 × DBN9004 | 100±0 | 3±1 |
| | NGM | 15±7 | 40±7 |
| VBC | DBN8205 × DBN8002 × DBN9004 | 100±0 | 3±1 |
| | NGM | 7±3 | 39±2 |
| SBL | DBN8205 × DBN8002 × DBN9004 | 100±0 | 3±1 |
| | NGM | 16±7 | 42±4 |
| SABW | DBN8205 × DBN8002 × DBN9004 | 100±0 | 3±1 |
| | NGM | 42±13 | 21±4 |
| FAW | DBN8205 × DBN8002 × DBN9004 | 100±0 | 3±1 |
| | NGM | 24±6 | 45±2 |
| BLAW | DBN8205 × DBN8002 × DBN9004 | 98±2 | 3±1 |
| | NGM | 8±3 | 38±5 |
| SPW | DBN8205 × DBN8002 × DBN9004 | 100±0 | 3±1 |
| | NGM | 15±7 | 25±8 |

The results show that the superimposed transgenic soybean event DBN8205 × DBN8002 × DBN9004 exhibits significantly higher mortality of tested insects than the NGM, and exhibits lower leaf damage ratio than the NGM against the above pests, indicating that the superimposed transgenic soybean event DBN8205 × DBN8002 × DBN9004 has good resistance against *Rachiplusia nu, Anticarsia gemmatalis, Chrisiodexys includes, Helicoverpa gelotopoeon, Spodoptera frugiperda, Spodoptera cosmioides, Helicoverpa zea.*

### Example 11: Detection of herbicide tolerance of the superimposed transgenic soybean event DBN8205 × DBN8002 × DBN9004

Nongda herbicide (41% glyphosate isopropyl-ammonium aqueous agent) and Basta herbicide (in which the active ingredient is 18% glufosinate ammonium aqueous agent) was selected for spray application in the experiment. Randomized block design with three replicates was employed. The district had an area of 15 m² (5 m × 3 m), row spacing of 60 cm and plant spacing of 25 cm, and one-meter isolation belt was set among districts for conventional cultivation and management. The superimposed transgenic soybean event DBN8205 × DBN8002 × DBN9004 was treated according to the following two manners: (1) no herbicide spray application, artificial grass control to removed effect of weeds on soybean growth; (2) spray application of Nongda herbicide at V3 leaf stage at a dose of 1680 g a.e./ha (a.e./ha means "active ingredient equivalent acid per hectare"), and then spray application of Nongda herbicide again at R2 stage (full flowering stage) at the same dose; (3) spray application of Basta herbicide at V3 leaf stage at a dose of 800 g a.i./ha (a.i./ha means "active ingredient per hectare"), and then spray application of Basta herbicide again at V6 stage at the same dose; (4) spray application of Basta herbicide at V3 leaf stage at a dose of 800 g a.i./ha, and then spray application of Nongda herbicide at R2 stage at a dose of 1680 g a.e./ha. Wild-type soybean plant (non-transgenic, NGM) was used as parallel control. It should be noted that if different amounts and dosage forms of glyphosate are converted into equivalent amounts of glyphosate acid, and different concentrations of glufosinate ammonium solutions are converted into the above equivalent effective ingredient glufosinate ammonium, they are all applicable to the following conclusions.

The investigation of phytotoxicity symptoms was respectively carried out at 1 week and 2 weeks after application, and the yields of the districts were determined at the time of harvest. The levels of phytotoxicity symptoms are shown in Table 14 of example 7. The herbicide tolerance of the transformation event was assessed by using the herbicide damage rate as an indicator. In particular, the herbicide damage rate (%) = Σ (number of plants of the same damage level × level number) / (total number of plants × highest level), wherein the herbicide damage rate includes the damage rate of glyphosate and the damage rate of glufosinate ammonium, and was determined according to the result of phytotoxicity investigation two weeks after glyphosate or glufosinate ammonium treatment. The soybean yield of each district was obtained by weighing the total yield (by weight) of soybean grains of 3 rows in the middle of each district. The yield difference among different treatments was measured as percentage of yield. The percentage of yield (%) = yield with spray application / yield without spray application. Results of herbicide tolerance of the superimposed transgenic soybean event DBN8205 × DBN8002 × DBN9004 and the soybean yield are shown in Table 20.

**Table 20: Results of herbicide tolerance of the superimposed transgenic soybean event DBN8205 × DBN8002 × DBN9004 and the yield test**

| Program/plant | DBN8205 × DBN8002 × DBN9004 | NGM |
|---|---|---|
| herbicide damage rate (%) (without spray application) | 0 | 0 |
| glyphosate damage rate (%) | 0 | 100 |
| glufosinate ammonium damage rate (%) | 0 | 100 |
| (glufosinate ammonium+glyphosate) damage rate | 0 | 100 |
| (%) | | |
| Percentage of yield (%) (glyphosate) | 103.5±9.7 | 0 |
| Percentage of yield (%) (glufosinate ammonium) | 102.8±8.6 | 0 |
| Percentage of yield (%) (glufosinate ammonium + glyphosate) | 101.4±9.5 | 0 |

The results show that, in terms of the herbicide (glyphosate and glufosinate ammonium) damage rate: 1) the damage rate of the superimposed transgenic soybean event DBN8205 × DBN8002 × DBN9004 treated with glyphosate herbicide (1680 g a.e. /ha) is basically 0; 2) the damage rate of the superimposed transgenic soybean event DBN8205 × DBN8002 × DBN9004 treated with glufosinate ammonium herbicide (800 g a.i./ha) is also basically 0; 3) the damage rate of the superimposed transgenic soybean event DBN8205 × DBN8002 × DBN9004 treated with glufosinate ammonium herbicide (800 g a.i./ha) and glyphosate herbicide (1680 g a.e. /ha) is also basically 0. Thus, the superimposed transgenic soybean event DBN8205 × DBN8002 × DBN9004 has good herbicide (glyphosate and glufosinate ammonium) tolerance.

In terms of the yield, comparing to the treatment without spray application, the superimposed transgenic soybean event DBN8205 × DBN8002 × DBN9004 treated with spray application of glyphosate herbicide (1680 g a.e. /ha), glufosinate ammonium herbicide (800 g a.i./ha) and glufosinate ammonium herbicide (800 g a.i./ha) + glyphosate herbicide (1680 g a.e. /ha) has a yield without significant difference. Thus, this further shows that the superimposed transgenic soybean event DBN8205 × DBN8002 × DBN9004 has good herbicide (glyphosate and glufosinate ammonium) tolerance.

### Example 11

Agricultural products or commodities can be produced from soybean plants comprising the transgenic soybean event DBN8205 or soybean plants comprising the transgenic soybean event DBN8205 and at least one transgenic soybean event different from transgenic soybean event DBN8205. If sufficient expression is detected in the agricultural products or commodities, they are expected to contain the nucleotide sequences that can be diagnostic for the presence of the transgenic soybean event DBN8205 materials in the agricultural products or commodities. The agricultural products or commodities include, but are not limited to, soybean cakes, powders and oils, specifically lecithin, fatty acids, glycerol, sterols, edible oils, defatted soy flakes, including defatted and baked soy flours, soy milk clots, bean curd, soy protein concentrates, isolated soy proteins, hydrolyzed vegetable proteins, organized soy proteins, soy protein fibers, and any other foods intended for consumption as a food source by an animal, etc. Nucleic acid detection methods and/or kits based on probes or primer pairs can be developed to detect nucleotide sequences derived from the transgenic soybean event DBN8205 in a biological sample, such as those shown in SEQ ID NO: 1 or SEQ ID NO: 2, wherein the probe sequence or primer sequence is selected from the sequences as shown in SEQ ID NO: 1, SEQ ID NO: 2, SEQ ID NO: 3, SEQ ID NO: 4 and SEQ ID NO: 5 or portions thereof, so as to diagnose the presence of the transgenic soybean event DBN8205.

In conclusion, the transgenic soybean event DBN8205 of the present invention has good resistance against *Lepidoptera* insects and high tolerance to glufosinate ammonium herbicide without compromising other agronomic traits and yields of the plant. Additionally, the detection methods of the present invention can accurately and rapidly identify whether a biological sample contains DNA molecules of the transgenic soybean event DBN8205.

The seeds corresponding to the transgenic soybean event DBN8205 were deposited under the Budapest Treaty at the General Microbiological Center of China Microbiological Culture Collection Management Committee (CGMCC for short, Address: No.3, No.1 Precinct, Beichen West Road, Chaoyang District, Beijing, Institute of Microbiology, Academy of Sciences of China, zip code 100101) on December 27, 2021, of which the classified nomenclature is soybean (*Glycine max*), the deposit status is viable, and the accession number is CGMCC No. 45071. The deposit will be deposited in the depository for 30 years.

At last, it should be noted that the above Examples are only used to illustrate the technical solutions of the present invention rather than limit the present invention. Although the present invention is described in detail with reference to the preferred Examples, those skilled in the art should understand that the technical solutions of the present invention could be modified or substituted equivalently without departing from the spirit and scope of the technical solutions of the present invention.

## Claims

1. A nucleic acid molecule having the following nucleic acid sequence, **characterized in that** the nucleic acid sequence comprises at least 11 consecutive nucleotides at positions 1-462 of SEQ ID NO: 3 or a complementary sequence thereof, and at least 11 consecutive nucleotides at positions 463-634 of SEQ ID NO: 3 or a complementary sequence thereof; and/or at least 11 consecutive nucleotides at positions 1-225 of SEQ ID NO: 4 or a complementary sequence thereof, and at least 11 consecutive nucleotides at positions 226-642 of SEQ ID NO: 4 or a complementary sequence thereof;
preferably, the nucleic acid sequence comprises 22-25 consecutive nucleotides at positions 1-462 of SEQ ID NO: 3 or a complementary sequence thereof, and 22-25 consecutive nucleotides at positions 463-634 of SEQ ID NO: 3 or a complementary sequence thereof; and/or 22-25 consecutive nucleotides at positions 1-225 of SEQ ID NO: 4 or a complementary sequence thereof, and 22-25 consecutive nucleotides at positions 226-642 of SEQ ID NO: 4 or a complementary sequence thereof;
preferably, the nucleic acid sequence comprises SEQ ID NO: 1 or a complementary sequence thereof, and/or SEQ ID NO: 2 or a complementary sequence thereof;
preferably, the nucleic acid sequence comprises SEQ ID NO: 3 or a complementary sequence thereof, and/or SEQ ID NO: 4 or a complementary sequence thereof.

2. The nucleic acid molecule according to claim 1, **characterized in that** the nucleic acid sequence comprises SEQ ID NO: 5 or a complementary sequence thereof.

3. A method for detecting the presence of the DNA of the transgenic soybean event DBN8205 in a sample, **characterized in** comprising:
- contacting the sample to be detected with at least two primers for amplifying a target amplification product in a nucleic acid amplification reaction;
- performing the nucleic acid amplification reaction; and
- detecting the presence of the target amplification product;
wherein the target amplification product comprises the nucleic acid sequence according to claim 1 or 2; preferably, the target amplification product comprises SEQ ID NO: 1 or a complementary sequence thereof, SEQ ID NO: 2 or a complementary sequence thereof, SEQ ID NO: 6 or a complementary sequence thereof, and/or SEQ ID NO: 7 or a complementary sequence thereof.

4. The method for detecting the presence of the DNA of the transgenic soybean event DBN8205 in a sample according to claim 3, **characterized in that** the two primers comprise SEQ ID NO: 8 and SEQ ID NO: 9, SEQ ID NO: 10 and SEQ ID NO: 11, or the complementary sequences of SEQ ID NO: 1 and SEQ ID NO: 2.

5. A method for detecting the presence of the DNA of the transgenic soybean event DBN8205 in a sample, **characterized in** comprising:
- contacting the sample to be detected with a probe, wherein the probe comprises the nucleic acid sequence according to claim 1; preferably, the probe comprises SEQ ID NO: 1 or a complementary sequence thereof, SEQ ID NO: 2 or a complementary sequence thereof, SEQ ID NO: 6 or a complementary sequence thereof, and/or SEQ ID NO: 7 or a complementary sequence thereof;
- hybridizing the sample to be detected with the probe under stringent hybridization conditions; and
- detecting the hybridization of the sample to be detected with the probe.

6. The method for detecting the presence of the DNA of the transgenic soybean event DBN8205 in a sample according to claim 5, **characterized in that** at least one of the probes is labeled with at least one fluorophore.

7. A method for detecting the presence of the DNA of the transgenic soybean event DBN8205 in a sample, **characterized in** comprising:
- contacting the sample to be detected with a marker nucleic acid molecule, wherein the marker nucleic acid molecule comprises the nucleic acid sequence according to claim 1; preferably, the marker nucleic acid molecule comprises at least one sequence selected from the group consisting of SEQ ID NO: 1 or a complementary sequence thereof, SEQ ID NO: 2 or a complementary sequence thereof, and/or SEQ ID NOs: 6-11 or complementary sequences thereof;
- hybridizing the sample to be detected with the marker nucleic acid molecule under stringent hybridization conditions;
- detecting the hybridization of the sample to be detected with the marker nucleic acid molecule, and further performing a marker-assisted breeding analysis to determine whether the insect resistance and herbicide tolerance is genetically linked to the marker nucleic acid molecule.

8. A DNA detection kit, **characterized in** comprising at least one DNA molecule, wherein the DNA molecule comprises the nucleic acid sequence according to claim 1, and the DNA molecule can act as a DNA primer or a probe specific for the transgenic soybean event DBN8205 or progeny thereof; preferably, the DNA molecule comprises SEQ ID NO: 1 or a complementary sequence thereof, SEQ ID NO: 2 or a complementary sequence thereof, SEQ ID NO: 6 or a complementary sequence thereof, and/or SEQ ID NO: 7 or a complementary sequence thereof.

9. A plant cell or plant part, **characterized in that** the plant cell or plant part comprises a nucleic acid sequence encoding the insect-resistant Cry2Ab protein, a nucleic acid sequence encoding the insect-resistant Cry1Ac protein, a nucleic acid sequence encoding the glufosinate ammonium-tolerant PAT protein and a nucleic acid sequence of a specific region, wherein the nucleic acid sequence of the specific region comprises the sequence as set forth in SEQ ID NO: 1 and/or SEQ ID NO: 2; preferably, the nucleic acid sequence of the specific region comprises the sequence as set forth in SEQ ID NO: 3 and/or SEQ ID NO: 4;
preferably, the plant cell or plant part comprises transgenic soybean event DBN8205;
optionally, the plant cell or plant part further comprises at least one other transgenic soybean event different from transgenic soybean event DBN8205; preferably, the other transgenic soybean event is transgenic soybean event DBN9004 and/or transgenic soybean event DBN8002.

10. A method for protecting a soybean plant from insect invasion, **characterized in** comprising providing at least one transgenic soybean plant cell in the diet of a target insect, wherein the transgenic soybean plant cell comprises in its genome the sequence as set forth in SEQ ID NO: 1 and/or SEQ ID NO: 2; and ingestion of the transgenic soybean plant cell inhibits the target insect from further ingesting the transgenic soybean plant;
preferably, the transgenic soybean plant cell comprises in its genome the sequence as set forth in SEQ ID NO: 3 and/or SEQ ID NO: 4;
preferably, the transgenic soybean plant cell successively comprises in its genome SEQ ID NO: 1, the nucleic acid sequence at positions 866-12192 of SEQ ID NO: 5 and SEQ ID NO: 2, or comprises the sequence as set forth in SEQ ID NO: 5.

11. A method for protecting a soybean plant from damage caused by a herbicide or controlling weeds in a field in which a soybean plant is planted, **characterized in** comprising applying an effective amount of glufosinate ammonium herbicide into a field in which at least one transgenic soybean plant is planted, wherein the transgenic soybean plant comprises in its genome the sequence as set forth in SEQ ID NO: 1 and/or SEQ ID NO: 2, and the transgenic soybean plant is tolerant to glufosinate ammonium herbicide;
preferably, the transgenic soybean plant comprises in its genome the sequence as set forth in SEQ ID NO: 3 and/or SEQ ID NO: 4;
preferably, the transgenic soybean plant successively comprises in its genome SEQ ID NO: 1, the nucleic acid sequence at positions 866-12192 of SEQ ID NO: 5 and SEQ ID NO: 2, or comprises the sequence as set forth in SEQ ID NO: 5.

12. A method for breeding an insect-resistant and/or glufosinate ammonium herbicide-tolerant soybean plant, **characterized in** comprising:
- planting at least one soybean seed, wherein the soybean seed comprises in its genome a nucleic acid sequence encoding the insect-resistant Cry2Ab protein and/or a nucleic acid sequence encoding the insect-resistant Cry1Ac protein and/or a nucleic acid sequence encoding the glufosinate ammonium herbicide-tolerant PAT protein, and a nucleic acid sequence of a specific region, or the soybean seed comprises in its genome the nucleic acid sequence as set forth in SEQ ID NO: 5;
- growing the soybean seed into a soybean plant; and
- invading the soybean plant with a target insect, and/or spraying the soybean plant with an effective amount of glufosinate ammonium herbicide, and harvesting the plants with reduced plant damage as compared with other plants which do not comprise the nucleic acid sequence of the specific region;
wherein the nucleic acid sequence of the specific region comprises the sequence as set forth in SEQ ID NO: 1 and/or SEQ ID NO: 2; preferably, the nucleic acid sequence of the specific region comprises the sequence as set forth in SEQ ID NO: 3 and/or SEQ ID NO: 4.

13. A method for producing an insect-resistant and/or glufosinate ammonium herbicide-tolerant soybean plant, **characterized in** comprising:
- introducing a nucleic acid sequence encoding the insect-resistant Cry2Ab protein and/or a nucleic acid sequence encoding the insect-resistant Cry1Ac protein and/or a nucleic acid sequence encoding the glufosinate ammonium herbicide-tolerant PAT protein, and a nucleic acid sequence of a specific region comprised in the genome of a first soybean plant, or the nucleic acid sequence as set forth in SEQ ID NO: 5 in the genome of the first soybean plant, into a second soybean plant, thereby producing a plurality of progeny plants; and
- selecting the progeny plants comprising the nucleic acid sequence of the specific region, which are also insect-resistant and/or glufosinate ammonium herbicide-tolerant;
wherein the nucleic acid sequence of the specific region comprises the sequence as set forth in SEQ ID NO: 1 and/or SEQ ID NO: 2; preferably, the nucleic acid sequence of the specific region comprises the sequence as set forth in SEQ ID NO: 3 and/or SEQ ID NO: 4;
preferably, the method comprises
- sexually crossing a first soybean plant comprising the transgenic soybean event DBN8205 with a second soybean plant, thereby producing a plurality of progeny plants;
- selecting the progeny plants comprising the nucleic acid sequence of the specific region;
- invading the progeny plants with a target insect, and/or treating the progeny plants with glufosinate ammonium;
- selecting the progeny plants which are resistant to the target insect and/or tolerant to glufosinate ammonium herbicide.

14. An agricultural product or commodity derived from a soybean plant comprising the transgenic soybean event DBN8205, wherein the agricultural product or commodity is lecithin, fatty acids, glycerol, sterols, soy flakes, soy flours, soy proteins or their concentrates, soybean oils, soy protein fibers, soy milk clots or bean curd.

15. The agricultural product or commodity derived from a soybean plant comprising the transgenic soybean event DBN8205 according to claim 14, **characterized in that** the soybean plant further comprises at least one other transgenic soybean event different from transgenic soybean event DBN8205;
preferably, the other transgenic soybean event is transgenic soybean event DBN9004 and/or transgenic soybean event DBN8002.

16. A method for expanding the insect-resistant spectrum and/or the herbicide-tolerant range of a plant, wherein the transgenic soybean event DBN8205 is expressed in a plant together with at least one other transgenic soybean event different from transgenic soybean event DBN8205;
preferably, the other transgenic soybean event is transgenic soybean event DBN9004 and/or transgenic soybean event DBN8002.
